# EUROPEAN PATENT APPLICATION

(11) **EP 1 271 145 A1**
(43) Date of publication of application: **02.01.2003**
(21) Application number: 02013560.4
(22) Date of filing: 19.06.2002
(51) Int. Cl.: G01N 33/50, C12Q 1/68, C12N 5/06

(54) **Methods and cell populations for identifying and validating genomic targets, and for drug screening**

(30) Priority: 25.06.2001 US 300665 P; 09.05.2002 US 379083 P
(71) Applicant: Cardion AG, 40699 Erkrath (DE)
(72) Inventor: Ruhl, Michael, 40883 Ratingen (DE); Rüdiger, Manfred, 40789 Monheim (DE); Field, Loren J., Indianapolis Indiana 46260 (US); Abts, Harry, 50259 Pulheim (DE); Schiller, Hilmar, 50733 Köln (DE)
(74) Representative: Bösl, Raphael, Dr. rer. nat., Dipl.-Chem.

(57) **Abstract**

This invention relates to isolated, stem-cell derived cell populations and methods of use thereof. Methods of use include genomic target identification, validation of conventionally obtained genomic targets using e.g. expression profiling, positional cloning and/or transgenic animals, lineage-specific and/or transgene-specific drug screening and pharmacogenomics analysis.

## Description

### FIELD OF THE INVENTION

This invention relates to isolated cellular populations such as transgenic lineage-specific cell lines obtained from the differentiation of stem cells *in vitro* and methods of use thereof. Methods of use include functional identification and/or validation of genomic targets, and compound screening.

### BACKGROUND OF THE INVENTION

The elucidation of the human genome (Lander et al. (2001) Nature 409: 860-921; Venter et al. (2001) Science 291: 1304-1351) has created a foundation for comprehensive genome analysis, and potentially allows the identification of all human genes. Microarray expression analysis, DNA diagnostics and gene-driven drug discovery, among others, rely on knowledge of and access to this annotated genomic information. Extrapolating this information from the sequence databases can be done partially *in silico* by bioinformatic approaches using appropriate search algorithms designed to identify homologies to known genes or functional sequence elements. Due to the inherent limitations of these prediction-algorithms, only a minor portion of all genes represented in the human genome can be identified by this approach. In addition the physiologic function of these genes might not be drawn a priori from these data. Therefore, identification and functional characterization of expressed sequences in the relevant context of particular physiological or pathological situations is still a major unmet need in the pharmaceutical industry. Existing and emerging cell-based approaches require sample preparation, nucleic acid isolation, sequencing, incorporation into the proper cellular context, expression and functional analysis. With both the cell-based and bioinformatic approaches, the major task is the elucidation of the physiological and/or disease-related function of certain genes. The lack of appropriate systems for validation of proposed gene functions and subsequent discovery of drugs targeted against therapeutically relevant or disease related genes is the most relevant rate-limiting factor for functional genomics aiming to turn the crude sequence data obtained in the human genome project into new pharmaceuticals or diagnostic tools. Conventional genomics approaches have provided an oversupply of potential therapeutic targets. Due to the lack of appropriate systems for efficiently sorting through the potential targets and for identifying the relevant ones, target validation increasingly becomes a bottleneck for the discovery of new drugs based on traditional genomics approaches (Harvey (1999) TiPS, 20: 196-198).

In this regard, a finally validated target is a biomolecule or part of a biomolecule of a cell that is involved in a disease process, and that can act as a site for therapeutic intervention or leads to the identification of such a site (i.e. serves as a "pathfinder"). Targets include, for example, enzymes, regulatory proteins, receptors, and nucleic acid sequences. Target identification seeks to understand the biological components of a disease, and which of those are central to its progress. A target gene is a stretch of nucleic acids which constitutes the target itself or encodes a target as defined. A genomic target is a stretch of nucleic acids representing a gene being or encoding a target or a regulatory element capable of regulating the expression of a target gene. This includes, but is not limited to, promoter regions, enhancers and silencers.

Target validation involves the experimental identification of a previously unknown function and/or functional association of a potential target with a physiological and/or disease or other pathophysiological phenotype. A target or validated target is a biomolecule whose function and/or functional association has been identified/verified in a validation process. With the dramatically increasing number of potential targets (identified in genomics approaches) and compounds (generated for example by combinatorial chemistry), the pharmaceutical industry involved in genomics-based rational drug design needs to shorten the time between identification of a potential target and lead discovery. A key challenge for the industry is to identify and validate high-quality genomic targets faster and more reliably.

The ability to efficiently sort through the many potential targets to define a narrow and specific set of relevant genomic targets, or "validated genomic targets" is still lacking. Although the functional analysis of target genes is possible in gain or loss of function transgenic animals, their use has several shortcomings: It is expensive, time-consuming, and not suitable for the analysis of a large number of potential targets as well as the screening of drugs at larger scale. Moreover, the spectrum of existing transgenic models is limited, and the experimental effects observed are not necessarily correlated to the corresponding human disease. To meet the substantial need for fast, flexible, and scaleble target validation-tools, innovative cell-based assays that better mimic the general physiology or disease condition in the human setting are necessary (Gonzalez and Negulescu (1998) Current Opinion in Biotech 9: 624-631; Taylor et al. (2001) Curr Opin Biotechnol 12: 75-81) and there are growing attempts to integrate those assays in high-throughput screening (HTS) (Johnston and Johnston (2002) Drug Discov Today 7: 353-363). Cell-based assays in general have the advantage that the provided information has more clinical relevance than a mere biochemical assay where two compounds interact in solution, out of a cellular context. An illustrative example is the use of cell-based assays rather then biochemical assays for the validation of ion channel targets in HTS (Mattheakis and Savchenko (2001) Curr Opin Drug Discov Devel 4: 124-134; Numann and Negulescu (2001) Trends Cardiovasc Med 11: 54-59).

Once a potential target has been validated, compounds are screened for their ability to revert the target gene-mediated pathophysiological phenotype or to elicit the desired physiological response. The patent and other literature describe many methods of drug screening. For instance, U.S. Patent No. 6,238,869 describes a high-throughput assay system that detects the binding of a chemical to a particular nucleic acid molecule. This method, while able to screen large numbers of chemicals, provides no information regarding physiologic effect. Again, the availability of suitable cell-based assays would give more relevant information at this step of drug development.

In order to monitor the transcriptional activity of a target gene the activity of the corresponding promoter sequence coupled to a reporter gene could be used. Such a promoter-specific drug interaction approach is described in U.S. Patent No. 5,665,543. The method uses cells transfected with a marker gene under the control of a known promoter. Changes in expression of the marker gene in response to exposure to the chemical are correlated with the effect of the chemical on the promoter.

One major drawback with the described cell-based approaches is the complete lack or limited availability of appropriate cells that better mimic the general physiology or disease condition of the respective cell type of interest. In the ideal system, the cells utilized for genomic target validation and the subsequent drug discovery should mimic as closely as possible the corresponding fully-functioning cell in the body. However, most of the established cell lines that are currently used in drug discovery do not sufficiently mimic the relevant general physiology or disease condition. Furthermore, for certain cell types no suitable cell lines are available at all (e.g. cardiomyocytes). Cells directly isolated from mammalian tissues or organs ("Primary Cells") often lack the properties needed to establish cell lines that are suitable for target validation and/or drug discovery purposes. Shortcomings include in particular (i) limited availability of suitable donor tissue/organs (especially for human sources), (ii) batch-to-batch variability of primary cells and a change of cellular properties with continued growth *in vitro* leading to limited reproducibility of the drug discovery assays, (iii) limited capacity to proliferate *in vitro* restricting the potential scale of attendant drug discovery assays, and (iv) limited incorporation of recombinant DNA-constructs necessary for target validation or compound screening in the desired cell-based assay. Generation of larger quantities of a homogenous population of genetically modified cells to be used as cellular assay system is therefore difficult with conventional cell-based approaches in drug discovery.

The present invention provides productivity-enhancing methods and materials for the multiple steps involved in the discovery of drug candidates in general, and targeting on therapeutically relevant or disease associated genes in particular. Furthermore, the present invention provides the underlying cellular compositions, the underlying cell-based assays used for genomic target validation and finally the resulting assays for drug discovery. In particular, the invention encompasses the functional identification of novel targets as well as the functional characterization/validation of preexisting target candidates in a cellular context that mimics the relevant general physiology or disease condition for a successful drug discovery effort. The invention further provides cell-based assays for the identification of potential therapeutic compounds and optimization of lead compounds that elicit a certain therapeutic and/or phenotypic effect including, but not limited to, the transcriptional regulation of a particular target gene. The invention (in one aspect) is essentially an innovative cell-based assay system for multiple steps in rational drug discovery providing a physiologically/pathologically relevant cellular system. In particular, the invention overcomes several major limitations of conventional cell-based as well as animal model systems.

The present invention is further described in the following sections and in the claims.

### SUMMARY OF THE INVENTION

The present invention provides methods for identifying and/or validating genomic targets using cell populations derived *in vitro* from differentiating stem cells. Accordingly, in one embodiment, the invention provides the use of an isolated cellular population obtained as a subpopulation of cells from an *in vitro* differentiation of stem cells, in a genomic target identification and/or validation method. In certain forms, the invention provides a cellular assay for functional identification and/or validation of a potential genomic target, that comprises (a) providing an isolated cellular population having genetically-modified cells containing the potential genomic target operably coupled to a promoter effective for expression of the potential genomic target in the genetically-modified cells, wherein the isolated cellular population has been obtained as a subpopulation of cells from an *in vitro* differentiation of stem cells; and, (b) assessing the effect of expression of the potential genomic target in the isolated cell population.

Inventive genomic target validation methods may involve transfecting a multipotent cell, such as a stem cell, with a nucleic acid molecule representing a potential or yet unvalidated genomic target; differentiating the transfected multipotent cell into a specific cell lineage; isolating the specific cell lineage, e.g. from non-differentiated cells and/or other unwanted differentiated cells; and determining whether expression of the nucleic acid molecule results in a change of phenotype of the specific cell related to a disease/pathologic state or physiological state, wherein induction of such a change represents a validation of the genomic target.

Additional inventive genomic target identification methods may include transfecting a multipotent cell, such as a stem cell, with at least one marker useful for isolation of particular lineage-specific cells, and further with at least one additional nucleic acid molecule representing a potential genomic target; differentiating the transfected multipotent cell into a specific cell lineage; isolating the specific cell lineage, e.g. from non-differentiated cells or unwanted differentiated cells; comparing the phenotype of the isolated transfected cells to the phenotype of appropriate control cells, e.g. cells of the same specific lineage lacking the target nucleic acid and derived from a corresponding multipotent cell. In such methods, a nucleic acid could be identified as target based on its ability to alter the phenotype of the transfected cell in comparison to an appropriate control cell lacking the nucleic acid sequence.

Alternatively, the target identification could be based on challenging cells of the isolated cell lineage carrying the potential target nucleic acid, with stimuli known to influence the phenotype in an identifiable manner representing a pathologic situation; and comparing them to cells of the same lineage not carrying the potential target but challenged in the identical way, and/or comparing them to cells of the same lineage carrying the potential target but not challenged. Alterations of the phenotype between these two populations can be used to identify and/or validate the nucleic acid as a target.

Genomic target identification methods of the invention include those involving the transfection of sets of nucleic acids, such as nucleic acid libraries, into multipotent cells, such as stem cells, or into cell populations obtained from the *in vitro* differentiation of multipotent cells or stem cells. Accordingly, in other aspects, the invention provides methods for identifying a target nucleic acid, comprising the steps of (a) providing an isolated cellular population having cells genetically modified to contain differing ones of a set of nucleic acid molecules operably coupled to a promoter effective for expression of the nucleic acid molecule in the cells, wherein the isolated cellular population is obtained as a subpopulation of cells from an *in vitro* differentiation of multipotent or stem cells; and (b) assessing the isolated cellular population to identify a target nucleic acid. The nucleic acid library used in such methods may, for example, be a genomic library, a sense cDNA library, an antisense cDNA library, a mixed sense/antisense library, or an RNA interference (RNAi) library consisting of small interfering RNAs (siRNAs) (Paddison et al. (2002) PNAS: 1443-1448; Tuschl (2002) Nature Biotech. 20: 446-448). The target identification can be based on (cellular) assays appropriate to monitor the phenotypic changes as defined above. Alternatively, enrichment or depletion of particular nucleic acids associated with a particular phenotype could be monitored by differential hybridization of these sequence or sequences to the immobilized members of the library used. For this identification of target sequences by hybridization, immobilization of the library can be achieved upon DNA-chips.

The invention further encompasses a method of determining an activity (e.g. a physiologically or pathologically relevant activity) of a certain nucleic acid molecule (in sense or antisense orientation) in a specific cell type by (a) transfecting a multipotent cell, such as a stem cell, with the nucleic acid molecule (alone or in combination with one or more additional nucleic acid molecules); (b) differentiating the transfected multipotent cell into a cell lineage; and (c) comparing a cell derived from a transfected stem cell or multipotent cell to a cell derived from a corresponding non-transfected multipotent cell to determine a change (especially a phenotypic change) in the transfected cell, where the change in the transfected cell indicates the relevant physiologic or pathologic or other activity of the nucleic acid molecule in the cell lineage.

In other aspects, the invention provides methods for testing compounds for their effect on cells expressing a particular genomic target or other nucleic acid molecule. Thus, the invention also provides a method for assessing the effect of a compound, the method including the steps of (a) providing an isolated cellular population having genetically-modified cells containing an introduced nucleic acid molecule, such as a genomic target, operably coupled to a promoter effective for expression of the nucleic acid molecule in the genetically-modified cells, wherein the isolated cellular population has been obtained as a subpopulation of cells from an *in vitro* differentiation of multipotent or stem cells; (b) contacting the isolated cellular population with the compound; and (c) assessing an effect of the compound on cells of the isolated cellular population. In this aspect of the invention, the introduced nucleic acid is generally one other than a selection marker used for isolation of the isolated cellular population, although such a selection marker may also be present. Such inventive methods may include screening for physiologic activity of a compound by transfecting a multipotent cell, such as a stem cell, with the nucleic acid molecule(s); differentiating the transfected multipotent cell into a specific cell lineage; isolating the specific cell lineage, e.g. from non-differentiated and/or unwanted differentiated cells; contacting the transgenic lineage-specific cell line with the compound; and, measuring or otherwise identifying an effect of the compound on the transgenic lineage-specific cell line in comparison to an untreated cell population of the same transgenic lineage-specific cell line, wherein a change in the phenotype of the compound-treated cells in comparison to the untreated control cells indicates a physiologic activity of the compound in cells of the particular cell lineage. In specific forms, such methods of the invention may involve screening a compound for its ability to revert the phenotype related to a genomic target to the normal phenotype represented by cells of the same specific lineage lacking the genomic target and derived from corresponding stem or multipotent cells.

For assessing in general the effect of a compound on a particular cell type an isolated cellular population can be used without the prior introduction of a target sequence. A method for assessing the effect of a compound may thus include the steps of: a) providing an isolated cellular population; said isolated cellular population obtained as a subpopulation of cells from an *in vitro* differentiation of stem cells; b) contacting the isolated cellular population with the compound; and c) assessing an effect of the compound on cells of the isolated cellular population by comparison to untreated cells of the same lineage. Such methods may be carried out for purposes including, but not limited to, toxicological or safety screenings for potential drugs. In another aspect of the invention, provided is a method for obtaining a substantially isolated cell line useful for the screens disclosed in the paragraph immediately above. In accordance with the invention, such a method may result in a substantially isolated lineage-specific cell line, transgenic for a (i.e. at least one) nucleic acid molecule to be studied, and may involve (a) transfecting a multipotent cell, such as a stem cell, with a nucleic acid molecule useful for selecting the lineage-specific cell population, and one or more additional nucleic acid molecules; (b) differentiating the transfected multipotent cell into a specific cell lineage; and (c) isolating cells of the specific cell lineage, e.g. from non-differentiated and unwanted differentiated cells of other lineages.

The invention also provides methods for assessing the effect of compounds on the transcriptional activity of a target gene, wherein the methods include the steps of (a) providing an isolated cellular population having genetically-modified cells containing an introduced nucleic acid encoding a reporter molecule operably coupled to a promoter for the target gene, the isolated cellular population obtained as a subpopulation of cells from an *in vitro* differentiation of multipotent cells; (b) contacting the isolated cellular population with the compound; and (c) assessing an effect of the compound on the level of activity of the reporter molecule. Such methods of the invention may involve screening compounds which transcriptionally regulate a target gene by producing a substantially isolated lineage-specific cell line, transgenic for a nucleic acid molecule effective for isolation of the desired cell lineage, and transgenic for a nucleic acid molecule (e.g. a reporter construct) including the promoter of the target gene controlling transcription of a reporter gene, the activity of which can be monitored, directly or indirectly e.g. by measurement of radioactivity, luminescence, fluorescence or adsorbtion, including but not limited to chloramphenicol acetyltransferase (CAT), β-galactosidase, luciferase (e.g. isolated from firefly or Renilla and modifications thereof), fluorescent proteins such as green fluorescence protein (GFP), red fluorescence protein (RFP), blue fluorescence protein (BFP) and derivatives thereof. Such an isolated cell population can be produced by transfecting a multipotent cell, such as a stem cell, with the nucleic acid molecules; differentiating the transfected stem cell into a specific cell lineage; and isolating the specific cell lineage, e.g. from non-differentiated cells and/or undesired differentiated cells. The transgenic lineage-specific cell line can be contacted with the compound; and an effect of the compound on the transgenic lineage-specific cell line can be measured based on the activity of the reporter gene. Thereby, a change in the activity of the reporter gene in response to a compound indicates physiologic activity of the compound concerning the transcriptional regulation of the target gene corresponding to the tested promoter.

Changes in phenotype that may be observed in genomic target identification and/or validation methods of the invention, or in compound screening methods of the invention, include alterations (induction /repression) of general physiologic processes such as gene expression, proliferation, apoptosis, necrosis, differentiation, migration, homing or attachment, cell-cell interactions, cell type specific functions like secretion, production of proteins, response to internal and/or external stimuli, contraction, electrical coupling, signal transmission, and the like.

Transfection protocols in methods of the invention can be applied to multipotent cells that are clonal, oligoclonal, or polyclonal. Correspondingly, the cell lineage differentiated therefrom can be clonal or polyclonal, and the cells used in the comparison can be these or clonal or polyclonal lines derived therefrom.

The present invention also encompasses stem cell populations, and cell populations derived therefrom, that are involved in the above-described methods, as well as methods for their manufacture.

Additional embodiments of the present invention will be apparent from the descriptions herein.

### BRIEF DESCRIPTION OF THE DRAWINGS

**Fig. 1. Map of the CT1-promoter based reporter vector**
   The promoterless luciferase reporter vector pGL3 was supplemented with a pGK-driven pac gene (puro^{r}) to confer puromycin resistance. To drive luciferase expression a 2357 bp long cardiotrophin 1 (CT-1) promoter-fragment was cloned into the multiple cloning site.
**Fig. 2. ES cell-derived genetically enhanced cardiomyocytes**
   A: Morphological characterization of a cluster of enriched differentiated cardiomyocytes. B: Western-blot analysis demonstrating the expression of β1-adrenergic receptors. Specific band for β1-receptor is indicated by the open arrow. CMs: cardiomyocyte at day 18 and 25 of differentiation, amH: adult mouse heart as positive control. Numbers on the right indicates position and size of marker bands in kDa.

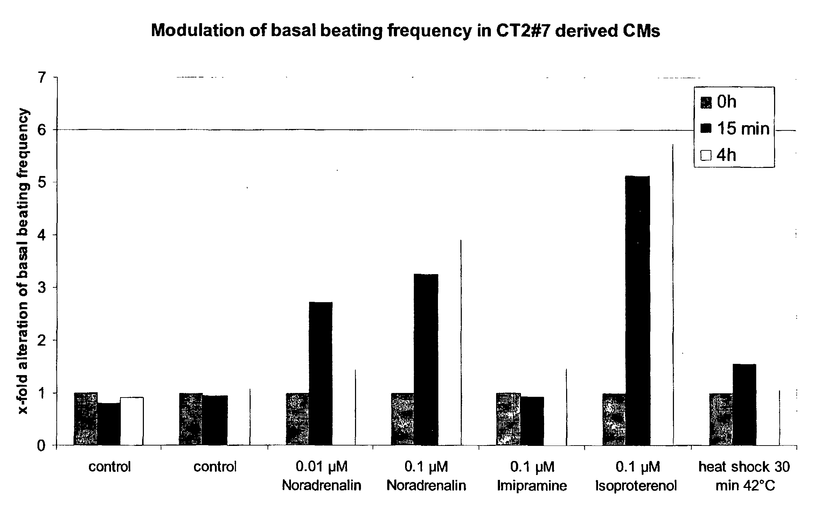

**Fig. 3. Modulation of beating frequency of ESC-derived CMs**
   Beating frequency was recorded for an individual cardiomyocyte cluster of each plate before stimulation (0h), 15 min after application of stimulus and at the end of the incubation period (4h).

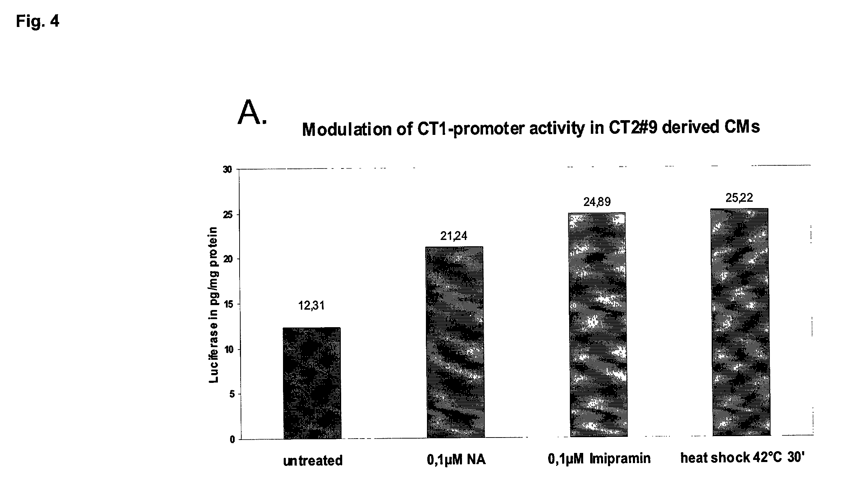

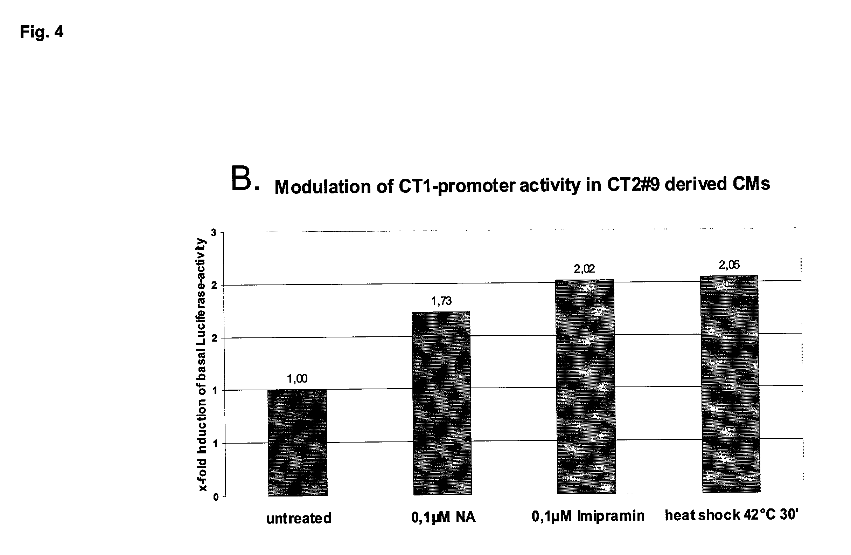

**Fig. 4. Modulation of CT1-promoter activity**
   ES cell-derived cardiomyocytes where incubated with the indicated compound for 4 h. Luciferase-activity was measured in cellular lysates and amount of Luciferase was corrected for total amount of protein.

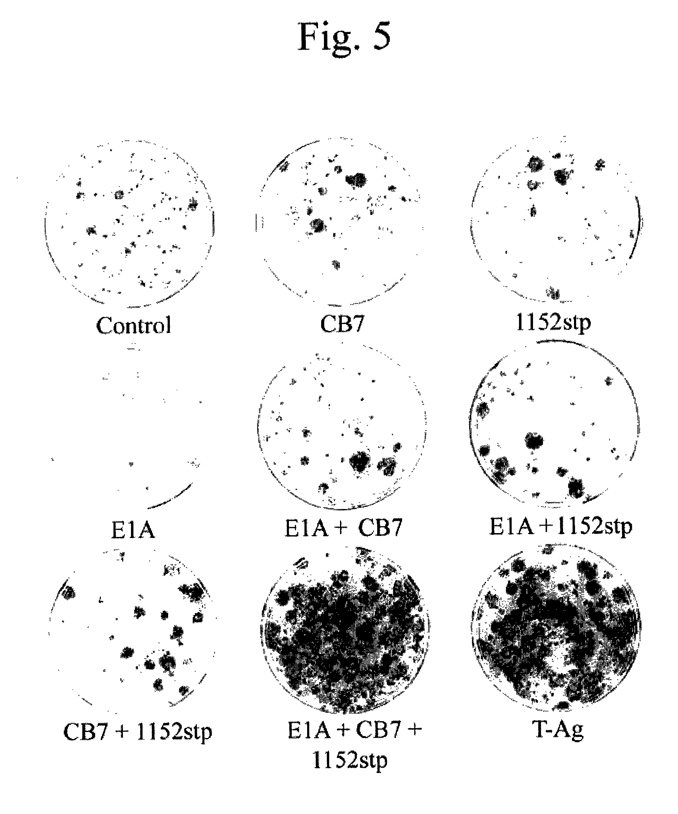

**Fig. 5. Co-expression of mutant p53 (CB7) and the pro-survival p193 mutant** **(1152stp) renders ES-derived cardiomyocytes responsive to E1A.**
   An ES-derived cardiomyocyte colony growth assay was used to monitor the effects of transgene expression on cardiomyocyte growth. All cultures were transfected with an MHC-neo^{r}/pGK-hygro^{r} transgene, as well as with MHC-promoted expression cassettes encoding the proteins indicated. After transfection and subsequent hygromycin selection, the ES cells were pooled and allowed to differentiate. Non-cardiomyocytes were removed by the addition of G418, and the yield of cardiomyocytes was directly visualized via PAS staining. The cultures were processed after 60 days of differentiation. Transfection efficiencies in parallel cultures were calculated by monitoring expression of a co-transfected CMV-ßGAL reporter gene at 48 hours post-transfection. βGAL activities (RLU/mg protein, x 10³) were: Control 1.2 ± 0.07; CB7 1.0 ± 0.04; 1152stp 1.1 ± 0.06; E1A 1.2 ± 0.01; CB7+E1A 1.3 ± 0.05; E1A+1152stp 1.2 ± 0.05; CB7+1152stp 1.0 ± 0.04; E1A+CB7+1152stp 1.2 ± 0.06; T-Ag 1.2 ± 0.02. There was no significant difference between groups by Kruskal-Wallis Nonparametric ANOVA test.

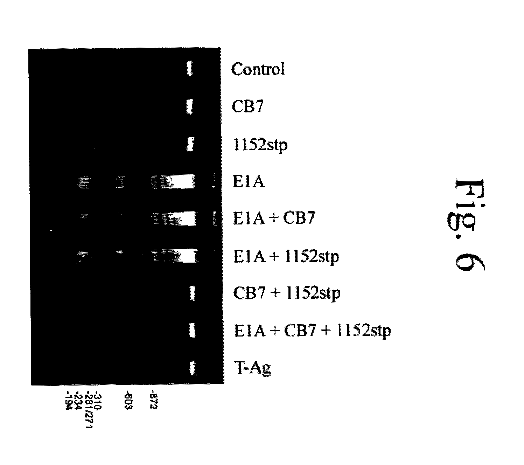

**Fig. 6. Inhibition of DNA fragmentation in ES-derived cardiomyocyte cultures co-expressing E1A + CB7 + 1152stp.**
   ES-derived cardiomyocyte cultures transfected with the various MHC-promoted expression cassettes were harvested after 15 days of differentiation. DNA prepared from the various cultures was analyzed by agarose gel electrophoresis. Marked internucleosomal DNA fragmentation was evident in ES-derived cardiomyocyte cultures expressing E1A, E1A + CB7, and E1A + 1152stp, but not in cultures expressing E1A + CB7 + 1152stp. DNA fragmentation was not detected in any of the other cultures analyzed.

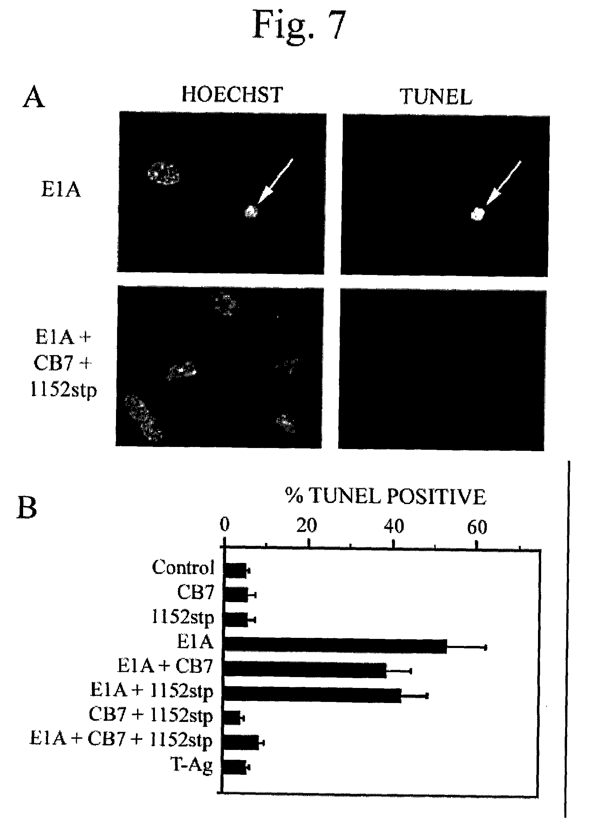

**Fig. 7. Decreased TUNEL positivity in ES-derived cardiomyocytes co-expressing E1A + CB7 + 1152stp.**
   Dispersed cell preparations were generated from ES-derived cardiomyocyte cultures transfected with the various MHC-promoted expression cassettes following 15 days of differentiation. The dispersed cells were replated on chamber slides and assessed for TUNEL positivity after an additional 24 hrs of culture. (A) Representative images of TUNEL reactivity (green signal) for ES-derived cardiomyocytes expressing E1A alone, or expressing E1A + CB7 + 1152stp are shown. Samples were counterstained with Hoechst 33342 to permit visualization of the nuclei. (B) Percentage of TUNEL positive cells in all cultures of ES-derived cardiomyocytes. High rates of TUNEL positivity were evident in cultures expressing E1A, E1A + CB7, and E1A + 1152stp, but not in cultures expressing E1A + CB7 + 1152stp. For each sample, a total of 300 cells were counted from 12 independent microscopic fields.

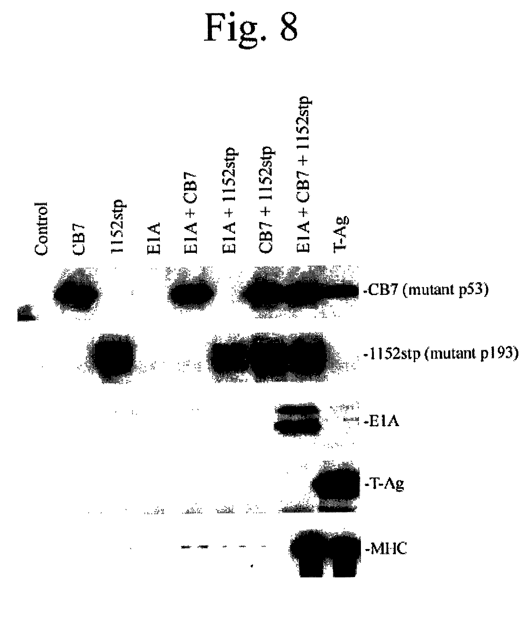

**Fig. 8. Confirmation of transgene expression in the ES-derived cardiomyocyte cultures.**
   Protein and RNA were prepared from ES-derived cardiomyocyte cultures transfected with the various MHC-promoted expression cassettes following 60 days of differentiation. 75 µg total protein was subjected to Western blot analysis to monitor CB7, E1A and T-Ag expression, and 10 µg total RNA was subjected to Northern blot analysis to monitor 1152stp expression. For MHC expression, 1% of the total protein present in the culture dishes was analyzed. Note that T-Ag expression stabilizes endogenous p53, which is also detected by the antibody used.

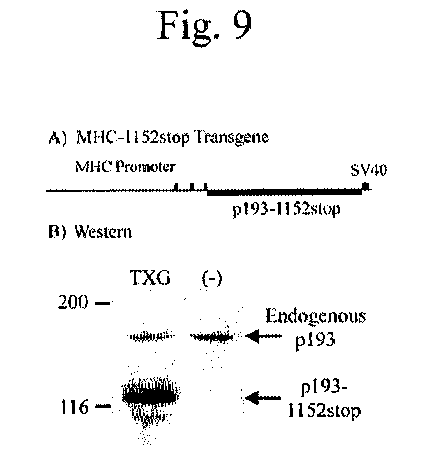

**Fig. 9. Generation and expression analysis of the MHC-1152stop transgenic mice.**
   (A) Schematic representation of the MHC-1152stp transgene. (B) Expression of 1152stop (1152stp) protein in the heart of a MHC-1152stp transgenic mouse (from MHC-1152stp line 9); protein from the heart of a non-transgenic littermate is shown as a negative control (-). Note that expression of the endogenous p193 protein is detectable in both the non-transgenic and transgenic hearts.

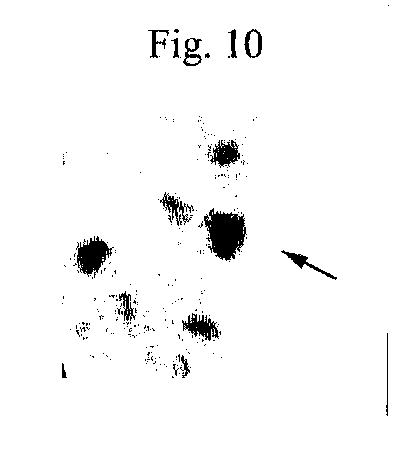

**Fig. 10. Example of X-GAL/ISEL co-staining to identify apoptotic cardiomyocytes.**
   For this study, an MHC-nLAC heart was cryopreserved, sectioned at 10 microns and sections stained with X-GAL to identify cardiomyocyte nuclei (cardiomyocyte nuclei stained blue due to the nuclear beta-galactosidase activity encoded by the MHC-nLAC transgene). The sections were then counter-stained using the Oncogene Research KLENOW-FragEL DNA Fragmentation Detection Kit and signal developed with biotinylated nucleotides and horseradish peroxidase (HRP) conjugated streptavidin. Cardiomyocyte apoptosis was identified by the presence of black HRP signal over blue nuclear staining (arrow indicates an ISEL-positive cardiomyocyte nuclei).

### DETAILED DESCRIPTION OF THE INVENTION

For the purposes of promoting an understanding of the principles of the invention, reference will now be made to certain of its embodiments and specific language will be used to describe the same. It will nevertheless be understood that no limitation of the scope of the invention is thereby intended, such alterations, further modifications and applications of the principles of the invention as described herein being contemplated as would normally occur to one skilled in the art to which the invention relates.

Certain terms used herein are defined as follows:
"Multipotent cells" - cells having the capacity to differentiate into more than one cell type.
"Stem cells" - cells that replicate as undifferentiated or lineage committed cells and have the potential to differentiate into at least one, preferably multiple, cell lineages.
"Nucleic acid molecule" - a polymeric form of nucleotides of any length, which contains deoxyribonucleotides, ribonucleotides, and analogs in any combination. Nucleic acid molecules as described herein may broadly include double-stranded, single-stranded, and triple-helical molecules. Unless otherwise specified or required, any embodiment of the invention described herein that is or involves a nucleic acid molecule encompasses both the double-stranded form and each of two complementary single-stranded forms known or predicted to make up the double stranded form of either the DNA, RNA or hybrid molecules. The following are non-limiting examples of nucleic acid molecules: a gene or gene fragment, exons, introns, mRNA, tRNA, rRNA, ribozymes, small interfering RNAs (siRNAs), cDNA, recombinant nucleic acid molecules, branched nucleic acid molecules, plasmids, vectors, isolated DNA of any sequence, isolated RNA of any sequence, nucleic acid probes, and primers. A nucleic acid molecule may comprise modified nucleotides, such as methylated nucleotides and nucleotide analogs, uracyl, other sugars and linking groups such as fluororibose and thioate, and nucleotide branches. The sequence of nucleotides may be interrupted by non-nucleotide components. A nucleic acid molecule may be further modified after polymerization, such as by conjugation with a labeling component. Other types of modifications included in this definition are caps, substitution of one or more of the naturally occurring nucleotides with an analog, and introduction of means for attaching the nucleic acid molecule to proteins, metal ions, labeling components, other nucleic acid molecules, or a solid support.
"Recombinant nucleic acid molecule" - a nucleic acid molecule of genomic, cDNA, semisynthetic, or synthetic origin which either does not occur in nature or is linked to another nucleic acid molecule in a non-natural arrangement. Typical such recombinant nucleic acid molecules are the result of genetic engineering involving *in vitro* transcription, enzyme modification (e.g. restriction digestion), amplification, and ligation.
"Vector" - a recombinant DNA or RNA plasmid or virus that comprises a heterologous nucleic acid molecule to be delivered into a target cell, either *in vitro* or *in vivo.* The heterologous nucleic acid molecule may comprise a sequence of interest for purposes of therapy, and may optionally be in the form of an expression cassette. As used herein, a vector need not be capable of replication in the ultimate target cell or subject. The term includes cloning vectors for the replication of a nucleic acid molecule, and expression vectors for translation of a nucleic acid molecule coding sequence. Also included are viral vectors, which comprise a nucleic acid molecule encapsidated or enveloped in a viral particle.
"Heterologous" - derived from a genotypically distinct entity from the rest of the entity to which it is being compared.
"Cell line", "cell culture", or "cell population" - bacterial, plant, insect or higher eukaryotic cells grown or maintained *in vitro.*
"Host cell" - a prokaryotic or eukaryotic cell that has been genetically altered, or is capable of being genetically altered by administration of an exogenous nucleic acid molecule, such as a recombinant plasmid or vector. When referring to genetically altered cells, the term refers both to the originally altered cell, and to the progeny thereof. The descendants of a cell may not be completely identical (either morphologically, genotypically, or phenotypically) to the parent cell. Once introduced, the exogenous nucleic acid can be maintained within the host cell as a non-integrated vector (such as a plasmid) or integrated at a random position into the host cell genome. In addition the insertion could be done in a site-specific manner using homologous recombination (Doetschman et al. (1987) Nature 330: 576-578). Homologous recombination could be used to exchange a complete endogenous gene or parts of it against a gene or parts of it, carrying a specific mutation or a polymorphism (SNP). Furthermore a gene could be inserted directly under the control of a endogenous target promoter (Li et al. (1998) Cur Biol. 8: 971-974). For flexible exchange of different candidate sequences the integration could be facilitated by the use of an introduced signal sequence for appropriate recombinases such as Cre, FLP or resolvase (Seibler et al. (1998) Biochem. 37: 6229-6234; Araki et al. (1997) NAR. 25: 868-872) or any other enzyme capable of recombining DNA by means of specific target sequences (such as loxP, or FRT).
"Signal peptide" or "leader sequence" - a short amino acid sequence that directs a newly synthesized protein through a cellular membrane.
"Isolated" - with reference to a nucleic acid molecule or a polypeptide, means that it is substantially free of the materials with which it is associated in its native environment. "Substantially free" means at least 50% free of these materials.
"Potential Target or Unvalidated Target" - a biomolecule of a cell that could in principle have a functional association with a physiologic or pathophysiologic process identified in a target screening. Potential targets include, for example, enzymes, regulatory proteins, receptors and nucleic acids. A potential target may become a validated target or may lead to another potential target that becomes a validated target.
"Target or Validated Target" - a biomolecule of a cell that is confirmed as having a functional association with a physiologic process and/or disease or other pathophysiologic process, and that is confirmed to lead to a phenotypic change in the cell when modulated.
"Target Screening" - a process by which potential targets are identified from the entirety of all cellular biomolecules.
"Target Validation" - a process by which validated targets are sorted from among potential targets.
"Genomic target" - a nucleic acid molecule representing a gene being or encoding a target (potential or validated) or a regulatory element capable of regulating the expression of a gene for a target (potential or validated). Regulatory elements include, but are not limited to, promoter regions, enhancers and silencers.
"Unvalidated" - with reference to a target or a genomic target, means that the functional association with a physiological and/or pathophysiolgical phenotypic change is unknown. "Unknown", in this context, may be understood to describe a nucleic acid molecule or biomolecule (i) with no apparent homology to any nucleic acid molecule or biomolecule with a previously identified function; or (ii) with a function predicted from sequence homology to a known gene or biomolecule, but where that function has not been confirmed by other means; or (iii) with a previously known function, but which also has at least one function which has yet to be identified.

As disclosed above, the present invention provides methods for identifying and for validating genomic targets utilizing cells derived from the differentiation of stem or other multipotent cells *in vitro.* The invention also provides such cells themselves, methods for preparing such cells containing the genomic targets, and methods of using such cells to screen compounds for their effect on cells expressing genomic targets, or for their effect on the transcriptional activity of promoters of genomic targets.

Methods of the invention employ cell populations obtained from the differentiation of stem or multipotent cells *in vitro.* In this regard, the stem or multipotent cell can be any known in the art. Suitable stem cells include, but are not limited to, embryonic stem cells, adult stem cells, neural stem cells, hematopoietic stem cells, mesenchymal stem cells, peripheral blood stem cells and cardiac stem cells. Preferably, the stem cell is human. The quintessential stem cell is the embryonal stem cell (ES), as it has unlimited self-renewal and multipotent and / or pluripotent differentiation potential, thus possessing the capability of developing into any organ, tissue type or cell type. These cells can be derived from the inner cell mass of the blastocyst, or can be derived from the primordial germ cells from a post-implantation embryo (embryonal germ cells or EG cells). ES and EG cells have been derived from mice, and more recently also from non-human primates and humans. Evans et al. (1981) Nature 292: 154-156; Matsui et al. (1991) Nature 353: 750-2; Thomson et al. (1995) Proc. Natl. Acad. Sci. USA. 92: 7844-8; Thomson et al. (1998) Science 282: 1145-1147; and Shamblott et al. (1998) Proc. Natl. Acad. Sci. USA 95: 13726-31.

Another stem cell source are embryonal carcinoma cells (EC cells) which are derived from teratocarcinomas. Donovan and Gearhart (2001) Nature 414: 92-97; Thomson and Odorico (2000) TIBTECH 18: 53- 57.

Stem cells have been identified in most organs and tissues, including "adult stem cells" - i.e. cells (including cells commonly referred to as "progenitor cells") that can be derived from any source of adult tissue or organ and can replicate as undifferentiated or lineage committed cells and have the potential to differentiate into at least one, preferably multiple, cell lineages. The best characterized are the hematopoietic stem cells. The ultimate hematopoietic stem cell can give rise to any of the different types of terminally differentiated blood cells. This is a mesoderm-derived cell purified based on cell surface markers and functional characteristics. Hill et al. (1996) Exp. Hematol. 24: 936-43. Also well characterized is the neural stem cell and a number of mesenchymal stem cells derived from multiple sources. Flax et al. (1998) Nature Biotechnol. 16: 1033-1039; Clarke et al. (2000) Science 288: 1666; Bruder et al. (1997) J. Cell. Biochem. 64: 278-294; Yoo et al. (1998) J. Bone Joint Surg. Am. 80: 1745-1757; Makino et al. (1999) J. Clin. Invest. 103: 697-705; and Pittenger et al. (1999) Science 284: 143-147.

In accordance with the invention, the stem or multipotent cell used will be caused to differentiate to provide a desired population of cells that will be used for the genomic target identification and/or validation method as well as for compound screening. For example, such desired cells may be of mesodermal cell lineage, ectodermal cell lineage, or endodermal cell lineage. Specific mesodermal cell types include for example cells of connective tissue, bone, cartilage, muscle (including e.g. skeletal myocytes and cardiomyocytes), blood and blood vessel, lymphatic, notochord, pleura, pericardium, peritoneum, kidney, and gonad cells. Ectodermal cells include for example epidermal cells, glands of the skin, the nervous system, the external sense organs such as eyes and ears, mucus membranes such as the mouth and anus, and others. Endodermal cells include epithelial cells such as those of the pharynx, respiratory tract, digestive tract, bladder and urethra.

The specific, isolated cell lineage can include monopotent, multipotent or pluripotent progenitor cells, lineage-committed precursor cells or terminally-differentiated cells. Illustrative progenitor cells include, but are not limited to, CD34⁺ hematopoietic cells, pdx-expressing pancreatic precursor cells, oval liver cells and satellite cells. Illustrative terminally-differentiated cells include, but are not limited to, cardiomyocytes, neuronal cells, and pancreatic cells, in particular β-cells, and liver cells.

In methods of the invention, cells of the desired cellular lineage or other cell population will be capable of isolation from their mixture with other cells. For example, desired cells may have a positive selectable marker and expression of the marker can be used to facilitate isolation of the desired cells from other cells. Alternatively, cells other than those which are desired may include a negative selectable marker which is expressed in those cells but not in cells of the desired cell population. This negative selectable marker can then be used to selectively deplete a mixture of the cells of the undesired cell types.

The selectable marker utilized in the present invention may for example be a foreign gene, a cellular gene or a gene conferring resistance to antibiotics or other toxins, e.g., ampicillin, neomycin, hygromycin, puromycin or methotrexate. The selectable marker may alternatively be a growth promoting gene or a gene encoding a growth factor or growth factor receptor or signal-transducing molecule, or a molecule that blocks cell death. Such selection markers may complement auxotrophic deficiencies, or supply critical nutrients not available from complex media. These and other selectable marker mechanisms which provide to selected cells a relative survival advantage or disadvantage are contemplated within the present invention.

In other embodiments the selectable marker may be a cell surface antigen or other gene product allowing purification or depleting of expressing cells by physical separation means, for example by panning or fluorescence-activated cell sorting (FACS) or magnetic cell sorting (including but not limited to MACS, Dynabeads).

In preferred modes of carrying out the invention a selectable marker will be introduced into the host stem or multipotent cells prior to their differentiation to form the mixed cellular population from which the desired cellular population will be selected. The selectable marker can be introduced in any suitable manner by which the nucleic acid molecule is introduced into the cells, with or without prior coupling to carriers or complex formation, and with or without the involvement of specific receptors on the cells. Suitable genetic modification protocols include, for example, transfection; electroporation, lipofection; injection; ballistic gene transfer employing calcium chloride, rubidium chloride, calcium phosphate, DEAE-dextran, or other such substances; microprojectile bombardment; viral transfection; and non-viral transfection. The use of these and other methods is within the purview of those practiced in the field.

For example, vectors, such as cloning and expression vectors, may be used to genetically modify host cells (e.g. stem cells or their progeny) in accordance with the invention. Such vectors typically contain a selectable marker as discussed above (e.g. a gene encoding a protein necessary for survival or growth of a host cell transformed with the vector), although such a marker gene can be carried on another nucleic acid sequence co-introduced into the host cell. Vectors also typically contain a replication system recognized by the host.

Expression vectors generally are replicable nucleic acid molecule constructs that contain a nucleic acid molecule encoding a polypeptide or regulatory region of interest. The nucleic acid molecule can be operatively linked to suitable transcriptional controlling elements, such as promoters, enhancers and terminators. For expression (i.e., translation), one or more translational controlling elements are also usually required, such as ribosome binding sites, translation initiation sites, and stop codons. These controlling elements (transcriptional and translational) can be endogenous, or heterologous (i.e., derived from other genes or other organisms). A nucleic acid molecule sequence encoding a signal peptide can also be included to allow a polypeptide to cross or lodge in cell membranes or be secreted from the cell. A number of expression vectors suitable for expression in eukaryotic cells including yeast, avian, and mammalian cells are known in the art. One example of an expression vector is pcDNA3 (Invitrogen, San Diego, CA), in which transcription is driven by the cytomegalovirus (CMV) early promoter/enhancer. This vector also contains recognition sites for multiple restriction enzymes for insertion of a nucleic acid molecule of interest. Another example of an expression vector (system) is the baculovirus/insect system.

Suitable cloning vectors can be constructed according to standard techniques, or selected from a large number of cloning vectors available in the art. While the cloning vector selected may vary according to the host cell intended to be used, useful cloning vectors will generally have the ability to self-replicate, may possess a single target for a particular restriction endonuclease, or may carry marker genes. Suitable examples include plasmids and bacterial viruses, e.g., pUC18, mp18, mp19, pBR322, pMB9, ColE1, pCR1, RP4, phage DNAs, and shuttle vectors such as pSA3 and pAT28. These and other cloning vectors are available from commercial vendors such as BioRad, Stratagene, and Invitrogen.

As an alternative to genetic modification of stem cells or progeny thereof to incorporate selectable markers, the stem or multipotent cells may be obtained from transgenic animals or tissues thereof having the selectable marker introduced into their genetic complement. Both founder transgenic animals and progeny thereof carrying the selectable marker are considered useful in the present invention.

To enable isolation of a given cell lineage or population, the selectable marker can be operatively linked to a promoter which is differentially active in the cells of the desired cellular population, as compared to the undesired cells to be depleted. For example, the selectable marker can be operatively linked to a promoter element or other cis-regulatory element prior to introduction into the stem or multipotent cells or prior to creation of the transgenic animals. A wide variety of tissue and cell-specific promoters are known and can be utilized in the present invention. For example, suitable cell-specific promoters for obtaining cardiomyocytes cell populations include the promoter from alpha-myosin heavy chain (α-MHC) or beta-myosin heavy chain (β-MHC). Other illustrative promoter candidates and corresponding cell types in which the promoters are selectively active include ANF or MLC 2v (cardiac muscle), mCK (skeletal muscle), SM22-α (smooth muscle), NeuroD (neuronal), Albumin (hepatic), (ICAM-2) (vascular endothelial), cGATA-1 (erythroid). Still other candidate promoters are known to those skilled in the art and can be used in the present invention to enrich for a given cell population or lineage. General methodologies for such selection protocols applied to stem cell progeny are disclosed for example in WO 95/14079.

Nucleic acid molecules enabling the selection of a target cell type from other cells differentiating from the stem or multipotent cells can be introduced at the stem or multipotent cell level and/or at a differentiated level. The nucleic acid molecule may include a selectable marker gene operably linked to a tissue-specific promoter such as those promoters identified above or other similar promoters.

Thus, in embodiments of the invention, using a lineage-specific promoter, a transgenic lineage-specific cell line can be obtained by transfecting stem cells to introduce a marker gene enabling selection of a first cell lineage from other cell lineages resulting from the differentiation of the stem cells, causing the stem cells to differentiate *in vitro* and selecting said one cell lineage based on said marker gene *in vitro.*

Nucleic acid molecules enabling selection of transfected from non-transfected cells may also be used in such strategies. Thus, in embodiments of the invention involving a "double selection" protocol, stem cells are transfected to introduce (i) a first marker gene enabling selection of transfected stem cells from non-transfected stem cells and (ii) a second marker gene enabling selection of one cell lineage from other cell lineages. Transfected stem cells are then selected based on the first marker gene. The transfected (selected) stem cells are then caused to differentiate; and the desired cell lineage is selected based on the marker gene. More preferably, the first and second marker genes each confer resistance to an antibiotic. The post-transfection selection and the post-differentiation selection can thereby involve contacting a mixed cellular population with an antibiotic, thereby killing undesired cells but not cells expressing the corresponding antibiotic resistance gene.

In addition to or as an alternative to the above-discussed cell isolation methods involving the use of selection markers, chemical or biological agents may be added to a differentiating population of stem-cell-derived cells, in order to enrich the population in cells of certain type(s) relative to other type(s). For example, cardiomyocyte yield increases by addition of 10⁻⁹M retinoid acid (Wobus et al. (1997) J. Mol. Cell. Cardiol. 29: 1525-1539), DMSO (Monge et al. (1995) J. Biol. Chem. 270: 15385-15390) or a combination of TGF-β2 and retinoic acid (Slager et al. (1993) Dev. Gen. 14: 212-224). Induction of beta-like insulin-producing cells is enhanced upon addition of nicotinamide and bFGF (Lumelsky et al. (2001) Science 292: 1389-1394). Differentiation of neural cells increases when 10⁻⁷M retinoid acid (Rohwedel et al. (1999) Cells Tissues Organs 165: 190-202) or EGF and FGF-2 (Reubinoff et al. (2001) Nat. Biotech. 19: 1134-1140) are added. Endothelial differentiation is enhanced by a combination of VEGF, bFGF, EPO and IL-6 (Balconi et al. (2000) Arterioskler. Thromb. Vasc. Biol. 20: 1443-1451), whereas hepatocyte formation increases upon addition of aFGF, HGF, oncostatin M and dexamethasone (Hamazaki et al. (2001) FEBS Lett. 497: 15-19). PDGF-BB induces formation of smooth muscle cells (Yamashita et al. (2000) Nature 408: 92-96). Macrophage are induced by IL-3 (Wiles and Keller (1991) Development 111: 259-267), and cells of the erythroid lineage increase in number when adding IL-6 (Biesecker and Emerson (1993) Exp. Hematol. 21: 774-778). Osteoclast differentiation can be increased by 1α,25-dihydroxyvitamine D3 and dexamethasone (Yamane et al. (1997) Blood 90: 3516-3523) or a combination of osteoprotegerin-ligand and MCSF (Hayashi et al. (1998) Biochem. Cell. Biol. 76: 911-922). Osteoblast yield increases after addition of BMP-2 and compactin (Phillips et al. (2001) Biochem. Biophys. Res. Commun. 8: 478-484) or a mixture of ascorbic acid, beta-glycerophosphate, dexamethasone and retinoic acid (Buttery et al. (2001) Tissue Eng. 7: 89-99). Yield of other cell types may be influenced by addition of chemical or biological substances accordingly.

Genomic target identification and/or validation methods of the invention will involve the genetic modification of stem cells or progeny cells thereof to incorporate one or more introduced or heterologous nucleic acid molecules to be investigated. For example, genomic target identification methods will involve the genetic modification of a population of stem or progeny cells with a set of nucleic acid molecules (e.g. a library) potentially including genomic targets. An ultimate assessment of an isolated cell population therefrom, and phenotypically-altered cells within that population, may serve to both identify and functionally validate the genomic target(s). Genomic target validation methods may involve the genetic modification of the stem or progeny cells with a nucleic acid molecule that has already been identified as a potential genomic target, but which remains unvalidated within any cellular context, or in a specific cell lineage or population to be isolated studied in accordance with the invention.

Such genetic modifications to incorporate the nucleic acid molecule(s) to be investigated may be accomplished by any suitable method, including those specifically identified above, and others known in the art. Further, the genetic modification to incorporate the genomic target may be applied at the stem cell level, or after differentiation of the stem cells has commenced. In the latter case, the genetic modification may be applied prior to or after a desired cell lineage or population is isolated from other undesired cells such as undifferentiated cells and/or cells of undesired lineages. Additionally, the stem cells utilized in the invention may be clonal or polyclonal. Likewise, the cell populations differentiated and isolated therefrom may be clonal or polyclonal, and are preferably enriched in a desired cell lineage, for example constituted at least about 50% of cells of a given lineage. In some preferred forms of the invention, the isolated cell population will be constituted of at least about 80% cells of a given lineage, more preferably at least about 95%, and most preferably 99-100%.

The nucleic acid molecule(s) to be investigated may encode an unknown biomolecule(s), or a biomolecule(s) of unknown function. The nucleic acid molecule or biomolecule may, for example, be associated with information implicating the nucleic acid molecule or biomolecule as potentially having a role in a physiological and/or disease or other pathophysiological process. This information will often be derived from genomic target identification processes, for example from expression profiling screens in which the upregulation or downregulation of the expression a nucleic acid molecule is noted in cells representative of the disease process, or in which one or more modifications (for example, but not restricted to mutations or single nucleotide polymorphisms (SNPs)) occurs in the target nucleic acid molecule as compared to a corresponding wild-type molecule. Information implicating the nucleic acid molecule for study in connection with a disease process may also be derived, for instance, as a function predicted from sequence homology to a known nucleic acid molecule, or sequence homology among the respective encoded biomolecules.

In these regards, the disease process under study can be any known in the art including, but not limited to those related to, cancer, autoimmune diseases, cardiovascular diseases, renal diseases, liver diseases, diabetes, neuronal disorders, hematological diseases, immunodeficiencies and benign hyperproliferation. Methods of the invention will be carried out utilizing cell types in which the particular disease process under study is manifested, wherein the cells are derived from the *in vitro* differentiation of stem cells. Illustrative diseases of various types include, but are not limited to:
Cancers: astrocytoma, oligodendroglioma, ependymoma, medulloblastoma, primitive neural ectodermal tumor (PNET), pancreatic ductal adenocarcinoma, small and large cell lung adenocarcinomas, squamous cell carcinoma, basal cell carcinoma, bronchoalveolarcarcinoma, epithelial adenocarcinoma, and liver metastases thereof, hepatoma, cholangiocarcinoma, breast tumors such as ductal and lobular adenocarcinoma, squamous and adenocarcinomas of the uterine cervix, uterine and ovarian epithelial carcinomas, prostatic adenocarcinomas, transitional squamous cell carcinoma of the bladder, B and T cell lymphomas (nodular and diffuse) plasmacytoma, acute and chronic leukemias, lymphoma (Hodgkin and non-Hodgkin), malignant melanoma, soft tissue sarcomas and leiomyosarcomas.
Autoimmune diseases: systemic lupus erythematosus, rheumatoid arthritis, juvenile rheumatoid arthritis, diabetes, scleroderma, ankylosing spondylitis, Sjorgen's syndrome, Goodpasture's syndrome, Reiter's syndrome and vasculitis.
Cardiovascular diseases: myocardial infarction, coronary artery disease, systemic hypertension, pulmonary hypertension, hypertrophic cardiomyopathy, cardiac arrhythmias (including both ventricular and supraventricular), heart failure, dilatative cardiomyopathy and atherosclerosis.
Renal diseases: glomerulonephritis, chronic renal failure, acute renal failure, contrast media induced renal failure, drug-induced renal failure, diabetes-related renal failure, chronic and/or acute renal graft rejection or malfunction
Liver diseases: liver cirrhosis, and metabolic disorders.
Diabetes: diabetes type I and II, including LADA.
Neuronal disorders: subarachnoidal hemorrhage, stroke, Morbus Parkinson, and any psychosis with a physiological or pathophysiological metabolic correlate.
Hematological diseases: diseases of the erythropoetic system (any form of anemia), diseases of the leukopoeitic system (e.g. any form of granulocytosis, chronic myelotic leukemia, acute leukemia).
Immunodeficiencies: primary and secondary amyloidosis, and any autoimmune disease.
Benign hyperproliferation: prostate hyperplasia
It will be understood that these lists are illustrative, and that functional investigations of other diseases may also be undertaken in accordance with the present invention.

To identify and to validate genomic targets in accordance with the invention, cells genetically modified to contain the nucleic acid molecule(s) under investigation will be assessed for a measurable phenotypic change associated with the nucleic acid molecule(s) sufficient for the identification or validation process. Illustrative changes in phenotype that may be observed include alterations (induction/repression) of general physiologic processes such as gene expression, proliferation, apoptosis, necrosis, differentiation, migration, homing or attachment, cell-cell interactions, cell type specific functions like secretion, production of proteins, response to internal and/or external stimuli, contraction, electrical coupling, signal transmission, and the like. Such changes may be measured or otherwise observed using appropriate assays. Particularly in the case of genomic target identification methods using antisense cDNA library or RNAi library screens, enrichment or depletion of particular nucleic acid molecules associated with a particular phenotypic change will desirably be monitored by differential hybridization of these molecules to the immobilized members of the library used. For such methods, the members of the library used are preferably immobilized on a DNA-chip to facilitate the hybridization processing and readout.

The observation of a phenotypic change induced by the presence of a particular nucleic acid molecule will generally involve a comparison to appropriate control cells. Often, but not always, appropriate controls will be cells corresponding to those under study, except lacking the nucleic acid molecule. For example, in certain embodiments of the invention, the cell population under study may be obtained by introducing the nucleic acid molecule(s) into stem cells of a given constitution, differentiating those stem cells, and isolating the cell population under study (with cells containing the nucleic acid molecule(s)). In such cases, appropriate control cells will often be obtained by correspondingly differentiating stem cells of that same constitution, except lacking the nucleic acid molecule(s), and correspondingly isolating the cell population (with cells lacking the nucleic acid molecule(s)) to serve as a control.

Specific embodiments of the invention provide genomic target identification based upon enrichment or depletion of particular nucleic acid molecules associated with a particular phenotype. In this context, depletion or enrichment can be identified by differential hybridization of these nucleic acid molecules to the immobilized members of the library used. For these purposes, hybridization is preferentially performed on DNA-chips upon which members of the library are immobilized.

In certain embodiments, inventive methods for genomic target identification will utilize gene inactivation techniques such as the so-called "Technical Knock Out" (TKO) techniques. In TKO techniques, a cell population of a desired type (e.g. a specific cell lineage) is subjected to a stimulus known to invoke a phenotypic change in cells of the population. An antisense cDNA library is generated from the stimulated/treated cells and from the unstimulated/untreated cells. The combination of both resembles the complete antisense cDNA-library, and is used to transfect a population of stem cells, or progeny thereof. A corresponding, transfected cell population is isolated from the stem cells or progeny, using selection or other isolation techniques as described hereinabove. A first aliquot of the isolated population is subjected to the original stimulus, and a second aliquot is not. Nucleic acids (DNAs or RNAs) from these two aliquots are then respectively labeled with two different fluorescent dyes. The nucleic acids are then together hybridized to a DNA-chip containing a dot microarray of the complete antisense cDNA library or RNAi library. The relative binding of the dyes at each dot is determined. The amount of dye bound at each spot reflects the abundance of the gene fixed on that dot relative in the whole population. Accordingly, one can accurately determine whether there has been a change in relative abundance of individual molecules in the population. If there has been no change in abundance, then the ratio of the two dyes will be 1:1. If there has been a change in abundance, then the ratio of the two different dyes will also change. Genomic targets are thereby identified by their depletion or enrichment in response to the stimulus. For additional information about techniques involved in such approaches, reference can be made to U.S. Patent No. 6,057,111. In an alternative approach an RNA interference (RNAi) library consisting of small interfering RNAs (siRNAs) could be used.

In a typical genomics-based rational drug design process, validation of a genomic target is followed by the development of an assay for screening small molecules (e.g. molecules having a molecular weight of less than 10,000 daltons) or large molecules (e.g. polypeptides) for their ability to revert an altered phenotype caused by the genomic target to a normal phenotype, or otherwise modify the altered phenotype in a fashion that may be therapeutic or may be useful to advancing the understanding of a disease process. The present invention provides such screening processes using isolated, stem cell-derived cell populations having cells carrying a nucleic acid molecule including the genomic target. In this regard, the isolated cell population may be derived from the *in vitro* differentiation of stem cells as described herein. Likewise, the phenotypic change observed, the control cells used, and the disease under study, may take any of the forms described herein.

The present invention further provides a method of screening for compounds which transcriptionally regulate a target gene. Such methods of the invention include contacting an isolated, stem cell-derived cell population with the compounds, wherein cells of the population contain a promoter from the target gene operably coupled to a reporter gene. In this fashion, any alterations in the activity of the promoter in response to the compounds can be monitored by observation of the activity of the reporter molecule. Suitable reports include, but are not limited to, reporter genes the activity of which can be monitored directly or indirectly by measurement of radioactivity, luminescence, fluorescence or adsorbtion, including but not limited to chloramphenicol acetyltransferase (CAT), β-galactosidase, luciferase, fluorescent proteins such as green fluorescence protein (GFP), red fluorescence protein (RFP), blue fluorescence protein (BFP) and derivatives thereof. In an alternative approach, two independent promoters of two independent target genes could be coupled to different reporter genes and introduced in the host cell. Such a setting would allow the simulanous screening for drugs affecting both target genes based on the activity of the two reporter genes. Again, the isolated cell population used in the screen may be derived from the *in vitro* differentiation of stem cells as described herein, and the disease under study may be any of those described herein, or others.

Aspects of the present invention employ, unless otherwise indicated, conventional techniques of molecular biology (including recombinant techniques), microbiology, cell biology, biochemistry and immunology, which are within the skill of the art. Such techniques are explained fully in the literature, such as, "Molecular Cloning: A Laboratory Manual", second edition (Sambrook et al. 1989); "Oligonucleotide Synthesis" (Gait ed. 1984); "Animal Cell Culture" (Freshney ed. 1987); "Methods in Enzymology" (Academic Press, Inc.); "Handbook of Experimental Immunology" (Wei & C.C. Blackwell eds.); "Gene Transfer Vectors for Mammalian Cells" (Miller & Calos, eds. 1987); "Current Protocols in Molecular Biology" (Ausubel et al. eds. 1987); "PCR: The Polymerase Chain Reaction" (Mullis et al. eds. 1994); "Current Protocols in Immunology" (Coligan et al. eds. 1991). These techniques are applicable to the production of the nucleic acid molecules of the invention, and, as such, may be considered in making and practicing the invention.

For the purpose of promoting a further understanding of the invention, the following examples are provided. It will be understood that these examples illustrate, but do not limit, the invention.

### Example 1

### Generation of an isolated, stem cell-derived population and use thereof to screen compounds with effects on transcriptional activity of a genomic target

### A. Experimental Protocols

### 1.1 Introduction

Hypertrophy of the cardiac muscle is an important compensatory response of the heart to injury or to an increased demand for cardiac output. On the other hand, chronic cardiac hypertrophy can lead to pathological situations and eventually to heart failure (Braunwald et al. (2000) Circulation 102: IV-14-IV-23).

Analysis of the underlying complex alteration in the expression pattern in the cardiac myocytes have and will identify large number of potential target genes for pharmacological interventions (Sehl et al. Circulation. (2000) 101: 1990-1999; Friddle et al. PNAS 97: 6745-6750; Stanton et al. Circ Res. (2000) 86: 939-945). The resulting challenge is the generation of appropriate cellular screening systems on the basis of cells that represent as close as possible the primary phenotype. This is especially difficult for cardiomyocytes since the terminal differentiated cells have no proliferation capacity. Additionally adult cardiomycoytes are also difficult to transfect with recombinant DNA, which further decreases the chance to generate an assay-system. Even the use of cell lines is not a alternative in the case of cardiomyocytes since there are no appropriate lines available.

The self-renewal capacity of embryonic stem (ES) cells and their potential to differentiate in culture into a variety of cell types, including cardiac muscle cells (Wobus et al. (1991) Differentiation 48: 173-182; Klug et al. (1996) J. Clin. Invest. 98: 216-224) make them a potential basis of the desired cellular assay-systems. Especially the fact that ES cells can be genetically modified much easier than the differentiated cells offers new opportunities for the generation of target gene based assays. Since the differentiation of the ES cells results in a complex mixture of all possible cell types an efficient enrichment protocol would be necessary to obtain a homogeneous cardiomyocyte population. This enrichment could be done by introduction of a cell type specific marker (WO 95/14079, 26 May 1995; U.S. Patent Nos. 5,733,727 and 6,015,671) in addition to the reporter construct for the final screening.

In order to test the possibility to introduce these required recombinant DNA molecules we had to select a promoter of a target gene which is involved in the pathophysiology of the hypertrophic heart. It should be regulated by a know pharmacological relevant substance and it's mode of action should require cardiomyocyte-specific receptors.

Growing evidence demonstrates that humoral factors play important role in the pathogenesis and progression of ventricular remodeling and subsequent heart failure (Bueno et al. in "Cardiovascular genomics: new pathophysiological concepts" Kluwer, the Netherlands 2002). In addition to neurohumoral factors also cytokines appear to be involved in cardiac-related illnesses as congestive heart failure, ischemic heart disease, dilated cardiomyopathy, and septic cardiomyopathy.

Cardiotrophin-1 (CT-1) is a cytokine of the IL-6 superfamiliy and was initially isolated from mouse embryoid body based on its ability to induce cardiac myocyte hypertrophy *in vitro* (Pennica et al. (1995) Proc Natl Acad Sci. 92: 1142-1146). This correlation appears to be also relevant *in vivo* since CT-1 concentration is also upregulated in patients with heart failure (Talwar et al. (2002) Clin Sci. 102: 9-14; Ng et al. (2002) Clin Sci. 102: 411-416).

CT-1 binds to the gp130/leukemia inhibitory factor (LIF) receptor complex in cardiac myocytes and activates the JAK-STAT and MAP kinase pathways (Pennica et al. (1995) J Biol Chem 270: 10915-10922; Craig et al. (2001) J Biol Chem 276: 37621-37629).

CT-1 has been shown to be inducible by noradrenaline (norepinephrine) in neonatal cardiac myocytes (Funamoto et al. (2000) J Mol Cell Cardiol 32: 1275-1284).

In the following example we used the CT-1 promoter in order to demonstrate that a reporter construct driven by a target gene promoter can be efficiently integrated into the genome of ES cells in addition to a marker gene which facilitates the selection of cardiomyocytes. The genetically modified ES cells differentiate normally and allow the marker gene based enrichment of cardiomyocytes. These cardiomyocytes can be used to screen substances and other stimuli for their ability to modulate promoter activity of the target gene by means of reporter activity.

### 1.2 Generation of the reporter construct

As basis for the luciferase reporter construct the pGL3-Basic vector (Promega) with a promoter-less luciferase gene was chosen.

To facilitate the selection of cells with stable integrated reporter-constructs the pGL3-Basic was supplemented with the Puromycin-resistance gene pac (Puromycin-N-acetyl-transferase) from the vector pPUR (Clontech). The HindIII/BamHI pac-fragment was placed under control of the PGK-promoter isolated as SalI/HindIII fragment from the vector αMHC-neo^{r}/pGK-hygro^{r} (Klug et al. 1996) and inserted in the SalI/BamHI site of pGL3-Basic, thus, generating pGL3-pGK-puro^{r}.

Using genomic DNA (gDNA) from mouse ESC a 2357 bp long Cardiotrophin 1 (CT-1) promoter-fragment has been generated by PCR-amplification using a proof reading Taq enzyme-mix (High-Fidelity Mix from Roche) to reduce the introduction of mutations during the amplification. The 5'-CT primer was supplemented with a Mlu I- and the 3'-CT primer with a Xhol site in order to permit later cloning into the reporter vector. To enable efficient restriction digestion appropriate clamps have added to the 5'-ends of the primers.

The PCR-generated fragment covers the published noradrenaline response element between nucleotide positions -2174/-1540 (Funamoto et al. (2000) J Mol Cell Cardiol 32: 1275-1284). The amplified DNA-fragment was double digested with MluI and XhoI using standard protocols (Sambrook et al. (1989) "Molecular cloning" Cold Spring Harbor Laboratory Press, Cold Spring Harbor, N.Y.).

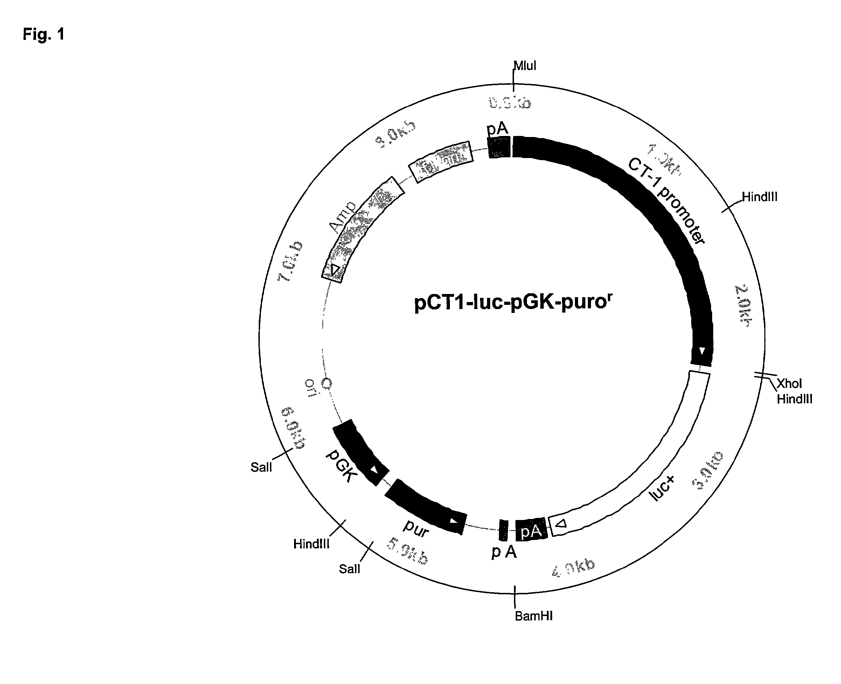

The digested fragment was purified by agarose-gel electrophoresis and cloned in the MluI and XhoI digested and gel-purified vector pGL3-pGK-puro^{r} resulting in the final CT1-promoter-based reporter construct pCT1-luc-pGK- puro^{r} (Fig. 1).

### 1.3 Generation of the gating construct

For gating on cardiomyocytes the vector αMHC-neo^{r}/pGK-hygro^{r} (Klug et al. (1996) J. Clin. Invest 98: 216-224) has been used. This gating-construct carries both an α-cardiac myosin heavy chain-aminoglycoside phosphotransferase (MHC-neo^{r}) and a phosphoglycerate kinase (pGK)-hygromycin resistance transgene in a common pBM20 vector backbone (Boehringer Mannheim, Indianapolis IN).

### 1.4 Generation of stable transfected mouse ESC clones

Undifferentiated J1-cells have been co-transfected with the gating construct αMHC-neo^{r}/pGK-hygro^{r} and the reporter construct pCT1-luc-pGK-puro^{r}.

### Electroporation

For electroporation a Gene Pulser II device (BioRad) was used. For co-electroporation the αMHC-neo^{r}/pGK-hygro^{r} was digested with VspI releasing a 1.4 kb backbone fragment and a 9.5 kb fragment containing the elements for clone selection and gating of cardiomyocytes. The reporter construct pGL3-CT1-luc pGK puro was linearized with XmnI. Restriction digest have been purified by ultrafiltration using Micron-PCR columns (Millipore). After sterile filtration the linear vector fragments have been used in equimolar amounts (total of 86.3 µg vector DNA) for co-electroporation of 5x10⁷ undifferentiated J1-cells.

### Selection of stable transfected clones

For the selection of stable transfected clones the transfected cells have been treated starting 24 h after electroporation for 7 days with hygromycin B (200 µg/ml) followed by 2 days with puromycin (2ug/ml).

Clonal cells where picked and separately expanded on a feeder layer of mitotically inactivated embryonic fibroblasts. During this expansion the cells are transferred from 96-well over 24-well to finally 6-well culture plates. At the last step the cells are cultiviated without feeder cells in the presence of hygromycin B (200 µg/ml).

### 1.5 Gating of cardiomyocytes

The gating of the ESC-derived cardiomyocytes is based on the previously described genetic enrichment protocol (Klug et al. (1996) J. Clin. Invest. 98: 216-224; WO 95/14079, 26 May 1995).

Briefly stable transfected ES cells were cultured in a bacteria dish. The resulting embryoid bodies (EBs) were distributed on cell culture dishes facilitating the attachment of the EBs. With appearance of spontaneous contractile activity (after subsequent 4-7 days) the cells were dissociated and replated on cell culture dishes. Following dissociation, G418 was added to the culture medium to enrich for cardiomyocytes. After 7 days selection plates contain 1-5x10⁵ cardiomyocytes.

### 1.6 Western-blot analysis

Gated cardiomyocytes have been lysed on the culture plates using reporter lysis buffer (Promega) supplemented with protease inhibitors (Roche). Equal amounts of total protein have been size separated in a 10% (polyacrylamide gel (PAA-gel) and transferred to PVDF-membrane by electroblotting. A prestained marker (broad range, BioRad) was use to estimate size of separated proteins. For detection of the β1-adrenergic receptor a specific rabbit polyclonal antibody (Oncogene) was used. A peroxidase labeled anti-rabbit antibody (Dianova) was used to detect the first antibody. LumiLight (PerkinElmer) reagent was used for detection and signals were visualized by exposure to X-ray film.

### 1.7 Screening of compounds

Gated cardiomyocytes where cultivated in 6 cm tissue culture dishes. Fresh medium was supplemented with the test compound in concentrations ranging from 0,01 - 10 µM. For heat shock treatment the cells where incubated at 42°C for 30 min. Mock treated cells served as control. Effects on the cardiomyocyte beating rate where recorded by manually counting the number of contractions during 15sec. Beating rates have been recorded before addition of substances and 15 min and 4h after addition. After 4h of incubation the cells where washed with PBS, collected in reporter lysis buffer (RLB, Promega) and stored at -80°C.

The Luciferase-activity in the thawed lysates was measured by flash-light Luciferase assay (Promega). In order to quantify Luciferase-activity a serial diluted recombinant Luciferase standard was analyzed in parallel. Values for Luciferase-activity where correct for the amount of total protein in the lysates measured by BCA-assay (Pierce).

### B. Results

### 1.8 Generation of double transfected cells

In order to generate a homogeneous cardiomyocyte population as cellular assay system we established a procedure which selects for integration of both, the gating construct as well as the reporter construct.

Undifferentiated J1 ES cells have been co-transfected via electroporation with the linearized gating- and reporter constructs. Stable transfected cells were selected by means of vector mediated resistance towards antibiotics. It was surprising to see that an initial selection with puromycin led to nearly elimination of all cells regardless the presence or absence of the vector, but that an initial selection with hygromycin B (200µg/ml) followed by a treatment with puromycin (2 µg/ml) resulted in efficient selection of double transfected cells. Clonal cells where picked and separately expanded as a first step towards the generation of large amounts of differentiated cells by the genetic enrichment protocol (gating). Presence of both constructs was confirmed by hygromycin treatment of the cells after the final expansion step and demonstration of integration of the luciferase reporter in the genomic DNA by PCR analysis (data not shown).

### 1.9 Gating of cardiomyocytes

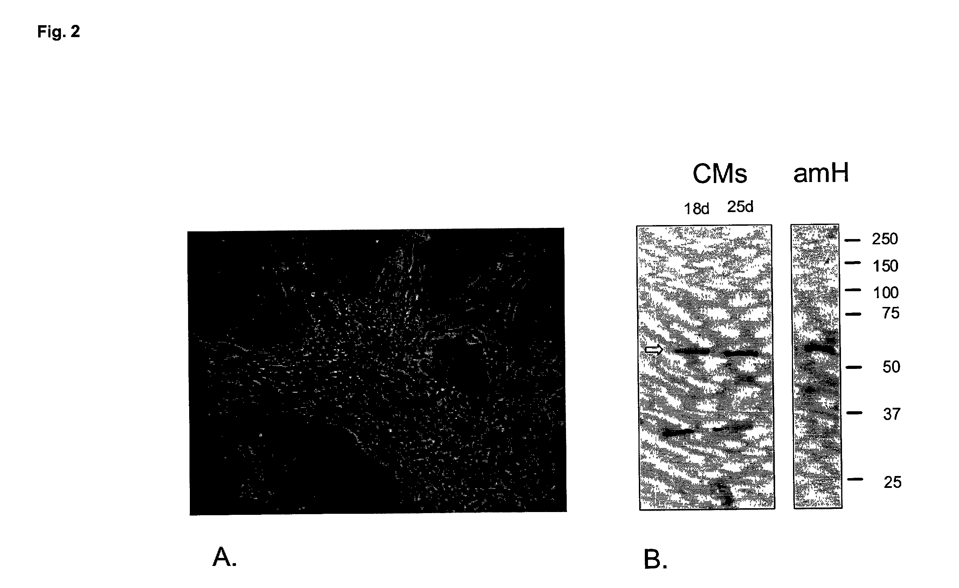

The gating of cardiomyocytes results in all cases in a substantial enrichment of cardiomyocytes. Dependent on the stringency of the gating procedure it is also possible to obtain pure cardiomyocyte populations. The cardiomyocytes are readily distinguishable from ES cells by their distinct morphology and due to their spontaneous contractile activity. Examples of ES-cell derived cardiomyocytes harboring the CT1-reporter construct are given in Fig. 2 A. To further document the cardiomyocyte nature of the gated cells the presence of β1-adrenergic receptors in the gated cells was demonstrated by Western-blot analysis (Fig. 2 B).

### 1.10 Response towards compounds

In order to modulate the expression of the cardiotrophin-1 (CT-1) gene we incubated the genetically engineered cardiomyocytes with noradrenaline, which has been reported to enhance transcriptional activity of the CT-1 promoter (Funamoto et al. (2000) J Mol Cell Cardiol 32: 1275-1284).

CT-1 treatment induces heat shock proteins such as hsp70 and hsp90. These CT-1 mediated induction of hsp protect cardiac cells from diverse stresses (Stephanou et al. (1998) J. Mol Cell Cardiol 30: 849-855). To investigate a direct effect of heat shock on the transcriptional activity of the CT-1 promoter we included also this stimulus in our analysis. Because CT-1 participates in processes leading to hypertrophy of the heart (Pennica et al. (1995) 92: 1142-1146; Railson et al. (2001) 33: 1209-1221) we analyzed also the effect of isoproterenol as known hypertrophic stimulus on the expression of CT-1. Furthermore we included imipramine in the testing as a compound not directly involved in the context of hypertrophic heart. Nevertheless, tricyclic antidepressants like imipramine are associated with cardiovascular side effects (Teschemacher et al. (1999) Br. J. Pharmacol. 128: 479-485).

### 1.11 Beating rate

Effects of noradrenaline are mediated via alpha and beta-adrenergic receptors, while isoproterenol effects are mediated specific via β-adrenergic receptors. Since β1 receptors play a pivotal role in mediating the beating rate, we analyzed changes in beating frequency of cardiomyocytes as a first indication of a response towards the tested compounds. The beating rate of a individual cluster per plate has been recorded before, 15 min after application of the stimulus and after 4h incubation. Fig.3 summarizes the data of such an analysis using cardiomyocytes 25 days after start of differentiation. As demonstrated, the treatment with noradrenaline leads to a dose dependent increase of this basal beating frequency. The positive chronotropic effect on the ES-cell derived cardiomyocytes was even more pronounced for the β-receptor specific isoproterenol. Due to the low starting beating frequency in some cases the x-fold increase is quite high. Nevertheless these elevated beating frequencies where still in the physiological range. Surprisingly we detected also an increase in beating frequencies after administration of 0.1 µM imipramine. The maximum increase with imipramine was in the same range as the maximum increase found after heat shock treatment.

### 1.12 Luciferase activity

The transcriptional activity of the CT1-promoter was analyzed by measurement of Luciferase activity in the cellular lysates. An example is shown in Fig. 4 A/B.

The functional activity of the generated reporter construct is documented by the basal activity of the promoter construct in the non-stimulated control cardiomyocytes. Following incubation for 4h with 0.1 µM noradrenaline this level increased 1.73-fold (Fig. 4 B). While this induction by noradrenaline was expected, the response towards the other applied stimuli has not been published before. It was therefore surprising to see that the incubation with 0.1 µM imipramine results also in a substantial increase of CT1-promoter activity which was more than 2-fold compared to the untreated control. In addition it was surprising to see, that a more than 2-fold induction of the basal activity was noticed also in response to heat shock (30min at 42°C). Thus, it appears that CT-1 does not only mediate its effects via activation of heat shock proteins (Stephanou et al. (1998) J. Mol Cell Cardiol 30: 849-855; Railson et al. (2001) 33: 1209-1221), but that CT-1 promoter-activity is itself inducible by heat shock.

### 1.13 Summary

The example demonstrates that in addition to the gating construct used for enrichment of cardiomyocytes an additional reporter construct, driven by a target gene promoter can be stably integrated in the genome of ES cells. The genetically modified ES cells form readily embryoid bodies from which the spontaneously differentiated cardiomyocytes could be efficiently enriched by the genetic gating protocol.

These cardiomyocytes express pharmacological relevant receptors and respond in a physiological way to pharmacological compounds (see also Wobus et al. (1991) Differentiation 48: 173-182; Wallukat and Wobus (1991) Rec Dev Toxicol, Suppl 14: 136-138). The effect of applied compounds on the transcriptional activity of a target promoter can be efficiently monitored by the activity of a reporter gene coupled to this promoter. In addition to reported effects on the transcriptional activity of the candidate (CT-1) promoter we were also able to discover formerly unreported effects of pharmacological substances and stress stimuli.

### Example 2

### Correlation of isolated, stem cell-derived population to in vivo cell population

### A. Experimental Methods:

### 2.1 Introduction

This example provides direct experimental evidence that isolated, stem cell-derived cell populations can be used as reliable models of cell behavior *in vivo* when expressing a functional nucleic acid molecule.

### 2.2. Recombinant DNA molecules.

The mouse α-cardiac myosin heavy chain (MHC) promoter (Gulick et al. (1991) J. Biol. Chem. 266:9180-9185) was used to generate expression constructs for embryonic stem cell-derived (ES-derived) cardiomyocyte colony growth assay and for the transgenic mouse models. To antagonize the p193 pathway, a cDNA encoding a C-terminal deleted p193 (amino acid residues #1 through #1152 (1152stp)) that encodes a pro-survival activity in the presence of MMS and similar agents (Tsai et al. (2000) J. Biol. Chem. 275:3239-3246) was subcloned downstream of the MHC promoter. For generation of a mutant p53 expression cassette, a p53 genomic clone from CB7 cells was utilized. Munroe et al. (1990) Mol. Cell Biol. 10: 3307-3313; and Lavigueur et al. (1989) Mol. Cell Biol. 9: 3982-3991. For generation of an E1A expression cassette, a wild-type E1A genomic clone was utilized which is alternatively spliced into 12s and 13s transcripts, but which lacks E1B. In all cases, standard recombinant protocols were employed, and the fidelity of the construct was confirmed by diagnostic nucleotide sequencing. Sambrook et al. (1989) "Molecular cloning" Cold Spring Harbor Laboratory Press, Cold Spring Harbor, N.Y.

### 2.3 ES cell culture conditions and cardiomyocyte colony growth assay.

The ES-derived cardiomyocyte growth assay was based on a previously described genetic enrichment protocol. Klug et al. (1996) J. Clin. Invest. 98: 216-224. Undifferentiated R1 ES cells were transfected via electroporation with a construct carrying both an α-cardiac myosin heavy chain-aminoglycoside phosphotransferase (MHC-neo^{r}) and a phosphoglycerate kinase (pGK)-hygromycin resistant transgene in a common pBM20 vector backbone (Boehringer Mannheim, Indianapolis IN). In some studies, the cells were co-transfected with multiple expression vectors. Transfected cells were selected by incubation in growth medium containing hygromycin B (200 µg/ml). After 7 days, the cultures were dissociated and 4 x 10⁶ cells were plated per 100 mm bacterial dish and cultured in growth medium lacking LIF. After 4 days of culture, the resulting embryoid bodies were collected and plated onto cell culture dishes. When spontaneous contractile activity was noticed (usually within 8-10 days of EB attachment), growth media supplemented with G418 (200 µg/ml) was added to eliminate the non-cardiomyocytes. Transfection efficiencies were calculated by monitoring expression of a co-transfected CMV-βGAL reporter gene at 48 hours post-transfection. Differences between groups were assessed by Kruskal-Wallis Nonparametric ANOVA test. To visualize cardiomyogenic induction, the plates were stained with Periodic Acid Schiff s (PAS) reagent.

### 2.4 Assessment of TUNEL activity in ES-derived cardiomyocytes.

For TUNEL analysis of DNA fragmentation in the ES-derived cardiomyocytes, dispersed cell preparations (obtained *via* trypsin treatment as described (Klug M.G., Soonpaa M.H., Koh G.Y., Field L.J. (1996) J. Clin. Invest. 98: 216-224) were plated onto fibronectin-coated chamber slides, fixed with 4% paraformaldehyde in PBS (pH 7.4) for 30 minutes at room temperature. The cells were permeablized with 0.1% Triton X-100 in 1% citric acid/PBS and processed for TUNEL staining using the In Situ Cell Death Detection kit (Roche, Indianapolis IN) according to the manufacturers' recommendations. Samples were then counter-stained with Hoechst 33342, and visualized via fluorescent microscopy.

### 2.5 Assessment of gene expression.

For Western blot analyses, cells were homogenized in NP40 buffer and the protein samples were displayed on polyacrylamide gels, transferred to nitrocellulose, and reacted with antibodies as described. Tsai et al. (2000) J. Biol. Chem. 275: 3239-3246; Laemmli (1970) Nature 227: 680-685; and Towbin et al. (1979) Proc. Natl. Acad. Sci. USA 76: 4350-4354. The antibodies used were: Anti-p53 PC35 (Oncogene, Cambridge MA), anti-ElA M73 (Santa Cruz Biotechnology Inc., Santa Cruz CA), anti-T-Ag PAB-416 (Oncogene) and anti-sarcomeric myosin MF20 (Developmental Studies Hybridoma Bank, University of Iowa, Iowa City IA). For Northern analysis, cells were homogenized with a polytron in 4.0 M guanidinium thiocyanate, 1% β-Mercaptoethanol, and total RNA purified as described. Chomczyski and Sacchi (1987) Anal. Biochem. 162: 156-159. Northern blots were performed as described previously. Tsai et al. 2000; and Sambrook et al. 1989.

### 2.6 Generation of transgenic mice.

To generate transgenic mice, an insert from the MHC-1152stp construct was isolated and microinjected into inbred C3HeB/FeJ (Jackson Laboratories, Bar Harbor, Maine) zygotes using standard methodologies (Hogan B., Costantini F. and Lacy L. (1986) "Manipulating the Mouse Embryo - A Laboratory Manual", Cold Spring Harbor Laboratory). The microinjected embryos were cultured *in vitro* to the two cell stage, and then reimplanted into pseudopregnant SW/Taconic (Taconic Farms, Germantown, New York) female mice. For all surgeries, mice were anesthetized with 2.5% Avertin (0.015 ml/g body weight IP, Fluka Biochemicals, Ronkomkoma, New York). All manipulations were performed according to NIH and Institutional Animal Care and Use Guidelines. Pups derived from the microinjected embryos were screened for the presence of the transgene using diagnostic PCR amplification as described (Steinhelper M.E., Cochrane K., Field L.J. (1990) Hypertension 16: 301-307). Positive animals were then used to establish lineages of transgenic mice by crossing with DBA/2J inbred mice.

### 2.7 Assessment of cardiomyocyte in situ end labeling (ISEL) activity in heart sections.

Cryosections (10 µm) were generated using standard histologic techniques. For ISEL analysis, cryosections were processed using the Oncogene Research KLENOW-FragEL DNA Fragmentation Detection Kit. Signal was developed with biotinylated nucleotides and horseradish peroxidase (HRP) conjugated streptavidin (Roche Molecular Biochemicals). Signal was generated by incubation with diaminobenzidine using standard techniques. Beta-galactosidase activity was detected in the same sections via X-GAL staining as described (Soonpaa M.H. et al. (1994) Science 264: 98-101).

### B. Results

### 2.8 Use of genetically enriched cell populations derived from differentiating ES cells to validate the pro-survival characteristics of the p193 1152stp molecule in cardiomyocytes.

The present invention maintains that genetically enriched cell populations derived from differentiating stem cells have the characteristics necessary for identification and/or validation of target genes of potential therapeutic utility. To directly test this, we sought to demonstrate that expression of the 1152stp molecule could block experimentally-induced apoptosis. Specifically, we sought to determine if expression of 1152stp alone, or in combination with expression of a dominant negative p53 transgene, would be sufficient to block E1A induced apoptosis in cardiomyocytes. Expression constructs encoding a dominant negative p53 (designated CB7, see Munroe et al. (1990) Mol. Cell Biol. 10: 3307-3313), the 1152stp and E1A under the regulation of the mouse MHC promoter were generated and used in an embryonic stem (ES) cell-derived cardiomyocyte colony growth assay. The cardiomyocyte colony growth assay was based on the previous observation that essentially pure cardiomyocyte cultures suitable for long term studies can easily be generated from differentiating ES cells that carry an MHC-neo^{r}/pGK-hygro^{r} transgene. Klug et al. (1996) J. Clin. Invest. 98:216-224. Undifferentiated ES cells were transfected with an MHC-neo^{r}/pGK-hygro^{r} transgene alone, or in combination with the MHC-CB7, MHC-1152stp and/or MHC-E1A transgenes. The presence of pGK-hygro^{r} sequences allowed for the selection of colonies of transfected, undifferentiated ES cells. The resulting colonies were pooled and used to generate embryoid bodies for *in vitro* differentiation. The presence of MHC-neo^{r} sequences allowed for enrichment of cardiomyocytes following *in vitro* differentiation: once cardiogenic induction was observed (as evidenced by the presence of spontaneous contractile activity), G418 was added to the cultures to eliminate non-myocytes. After 60 days of culture, the dishes were stained with Periodic acid Schiff's (PAS) reagent to visualize the cardiomyocytes (PAS stains glycogen containing cells intense violet).

The results are shown in Figure 5. The control dish depicts cardiomyocytes obtained by transfection with the MHC-neo^{r}/pGK-hygro^{r} transgene alone, and is indicative of the baseline rate of ES-derived cardiomyocyte growth under the conditions employed. Expression of CB7 or 1152stp did not have a marked impact on the yield of cardiomyocytes. In contrast, expression of E1A dramatically decreased cardiomyocyte yield, consistent with the pro-apoptotic activity of this molecule in cardiomyocytes as described by others. Co-expression of E1A and CB7 or E1A and 1152stp did not result in enhanced cardiomyocyte yield as compared to expression of CB7 or 1152stp alone, although the yield was increased as compared to cultures transfected with E1A only. In contrast, co-expression of E1A, CB7 and 1152stp markedly increased the yield of cardiomyocytes, with levels similar to those obtained from transfection with SV40 Large T Antigen (T-Ag). These results are consistent with the hypothesis that 1152stp encodes a pro-survival phenotype, and furthermore that co-expression of CB7 and 1152stp blocks cardiomyocyte apoptosis in response to E1A expression.

The pro-survival activity encoded by the 1152 molecule is further confirmed by assessment of DNA inter-nucleosomal fragmentation (Figure 6) and cardiomyocyte TUNEL activity (Figure 7); both are direct measures of apoptosis. As would be expected; apoptosis was apparent in cultures expressing E1A alone, E1A and CB7, or E1A and 1152stp. In contrast, no evidence for apoptosis was observed in cultures co-expressing E1A, CB7 and 1152stp. These data collectively indicate that co-expression of CB7 and 1152stp blocks E1A induced apoptosis in ES-derived cardiomyocytes. As a final control, transgene expression was confirmed for all these samples (Figure 8). Interestingly, these analyses confirm that cardiomyocytes expressing E1A were only viable if both CB7 and 1152stp are also expressed.

### 2.9 In vivo validation of a target identified using genetically enriched ES-derived cell populations.

A key aspect of the current invention is that targets identified and/or validated through the screening process in stem cell-derived cells will exhibit the same biologically relevant activity *in vivo.* The examples presented above suggest that the p193 1152stp molecule encodes an anti-apoptotic activity that antagonizes E1A-induced apoptotic death.

To directly provide *in vivo* validation of the *in vitro* testing, transgenic mice expressing a cDNA encoding 1152stp under the regulation of the MHC promoter were generated and subjected to pathophysiologic injury. The MHC-1152stp transgene is depicted in Figure 9A. This DNA was microinjected into zygotes, and the injected embryos were reimplanted into the oviducts of surrogate mothers and allowed to develop to term. Thirteen of the resulting animals carried the MHC-1152stp transgene. These animals were bred, and 7 independent transgenic lines were established. Transgene expression was confirmed for MHC-1152stp line #9 using Western blot analyses as is shown in Figure 9B. The monoclonal antibody used was raised against an epitope between amino acid residues #860 and #1150, and as such recognizes the endogenous full-length p193 protein as well as the 1152stp molecule. Note that expression of the endogenous p193 protein is detectable in both the non-transgenic and transgenic hearts. As expected, the 1152stp molecule is present in the MHC-1152stp line 9 animal (at approximately a 5- to 8-fold molar excess to the endogenous protein). Cardiac development in all of the transgenic mice was normal, with no obvious structural or functional changes.

Isoproterenol is a beta-adrenergic agonist that induces cardiomyocyte hypertrophy both *in vitro* and in vivo. Prolonged exposure to isoproterenol is cardiotoxic, inducing cardiomyocyte apoptosis via a mechanism similar to that encountered during the progression of heart failure. The MHC-1152stp mice were tested to determine if transgene expression was cardioprotective during isoproterenol infusion, and specifically to see if 1152stp expression could antagonize isoproterenol-induced cardiomyocyte apoptosis. To facilitate the analysis of cardiomyocyte apoptosis, MHC-1152stp mice were inter-crossed with MHC-nLAC mice (the presence of the MHC-nLAC transgene, which encodes nuclear localized beta-galactosidase activity exclusively in cardiomyocytes, greatly aided identification of cardiomyocyte nuclei (Soonpaa M.H., Field L.J. (1997) Am J Physiol. 272: H220-H226; Soonpaa M.H., Koh G.Y., Klug M.G. and Field L.J. (1994) Science 264: 98-101; Pasumarthi K.B., Nakajima H., Nakajima H.O., Jing S., Field L.J. (2000) Circ Res. 86: 1069-1077)).

Mice with a MHC-1152stp / MHC-nLAC genotype and a (-) / MHC-nLAC genotype were culled, and at 12 weeks of age were implanted with isoproterenol-filled osmotic mini-pumps, as described in several studies from our group (Soonpaa M.H. and Field L.J. (1994) American Journal of Physiology 266 (Heart and Circulatory Physiology 35): H1439-1445; Pasumarthi K.B., Nakajima H., Nakajima H.O., Jing S., Field L.J. (2000) Circ Res. 86: 1069-1077). After seven days of infusion, the increase in heart weight was the same between the control (i.e. (-) / MHC-nLAC) and experimental (MHC-1152stp / MHC-nLAC) mice). To monitor cardiomyocyte apoptosis, the hearts were cryoprotected, and sectioned at 10 microns. The sections were stained with X-GAL to identify cardiomyocyte nuclei (cardiomyocyte nuclei stained blue due to the nuclear betagal activity encoded by the MHC-nLAC transgene). The sections were also processed for ISEL to identify apoptotic nuclei; sections were processed using the Oncogene Research KLENOW-FragEL DNA Fragmentation Detection Kit and signal was developed with biotinylated nucleotides and horseradish peroxidase (HRP) conjugated streptavidin. Cardiomyocyte apoptosis was then calculated simply by determining the incidence of black HRP signal over blue nuclear staining; these analyses focused on cardiomyocytes residing in the ventricular septum, where the cardiotoxic effects of isoproterenol appear to be most pronounced. An example of ISEL positive cardiomyocytes using this assay is shown in Figure 10. In the absence of isoproterenol, the rate of cardiomyocyte ISEL positivity was exceedingly low in the (-)/MHC-nLAC mice (see Table 1). In contrast, nearly 0.1% of the cardiomyocyte nuclei in the ventricular septum of isoproterenol-treated (-) / MHC-nLAC mice were ISEL-positive. Only 0.004% of the cardiomyocyte nuclei in the ventricular septum of isoproterenol-treated MHC-1152stop / MHC-nLAC mice were ISEL-positive). These data provide direct validation that the biologic activity used as the primary screen in ES-derived cell lineages corresponds to that activity in the appropriate target cell *in vivo.*

**Table 1.**

| **Expression of 1152stp protects against isoproterenol-induced cardiomyocyte** **apoptosis *in vivo.*** | | | |
|---|---|---|---|
| ISEL Positive | Fold | | |
| Mouse/Treatment: | CM Nuclei: | % ISEL Positive | Protection* |
| MHC-nLAC/None (n = 5 mice) | 1 of 142,437 | < 0.0007% | N/A |
| | | | |
| MHC-nLAC/+ISO (n = 5 mice) | 35 of 35604 | 0.098% | N/A |
| | | | |
| MHC-1152stp + MHC-nLAC/+ISO (n = 4 mice) | 1 of 22,494 | 0.004% | 24.5 x |

| | | | |
|---|---|---|---|
| *: This indicates the degree to which expression of the MHC-1152stp transgene protects cardiomyocytes from isoproterenol-induced cardiomyocyte apoptosis. | | | |

All documents, publications and patent applications cited in this specification are incorporated herein by reference to the same extent as if each individual publication or patent application was specifically and individually indicated to be incorporated by reference. Although the foregoing invention has been described in some detail by way of illustration and example for purposes of clarity and understanding, it will be apparent to those skilled in the art that certain modifications may be practiced. Therefore, the description and examples should not be construed as limiting the scope of the invention, which is delineated by the appended claims.

## Claims

1. A cellular assay for identification and/or validation of a genomic target, comprising:
a) providing an isolated cellular population having genetically-modified cells containing said genomic target operably coupled to a promoter effective for expression of the genomic target in the genetically-modified cells, said isolated cellular population obtained as a subpopulation of cells from an *in vitro* differentiation of stem cells;
b) assessing the effect of expression of the genomic target in the isolated cell population.

2. A method for assessing a series of nucleic acid molecules for potential effect when expressed in an isolated cell population, said nucleic acid molecules in said series identified as potentially being associated with either a physiological or a disease process, said method comprising:
a) providing a plurality of isolated cellular populations each having genetically-modified cells containing one of said genomic target nucleic acid molecules operably coupled to a promoter effective for expression of the genomic target nucleic acid molecule in the genetically-modified cells, each of said isolated cellular populations obtained as subpopulation of cells from an *in vitro* differentiation of stem cells; and
b) assessing the effect of expression of the respective genomic target nucleic acid molecules in the respective isolated cell populations.

3. A method according to claim 1 or 2, wherein said effect includes a phenotypic change correlated to the expression of said genomic target nucleic acid molecule.

4. A method according to any of claims 1-3, wherein said isolated cellular population is predominantly comprised of a single cell type, and wherein said cells are mammalian cells.

5. A method according to any of claims 1-3, wherein said isolated cellular population is predominantly comprised of cells of a single type selected from cardiomyocytes, epithelial cells, mucosal cells, endothelial cells, smooth muscle cells, striated muscle cells, keratinocytes, fibroblasts, osteoclasts, osteblasts, chondroclasts, chondrocytes, pancreatic cells, hepatocytes, neuronal cells, glial cells, astrocytes, oligodendrocytes, hematopoietic cells, and kidney cells.

6. A method according any of claims 1-5, wherein said isolated cellular population is obtained by:
providing stem cells containing said genomic target nucleic acid molecule operably coupled to said promoter;
differentiating the stem cells; and
isolating the isolated cell population from cells differentiated from said stem cells.

7. A method according to any of claims 1-5, wherein said isolated cellular population is obtained by:
providing stem cells;
differentiating the stem cells;
isolating the isolated cell population from cells differentiated from said stem cells; and
genetically modifying said isolated cell population to contain said genomic target nucleic acid molecule operably coupled to said promoter.

8. A method according to any of claims 1-7, which is a genomic target identification method.

9. A method according to any of claims 1-6, which is a genomic target validation method.

10. A method for assessing the effect of a compound comprising the steps of:
a) providing an isolated cellular population having genetically-modified cells containing an introduced nucleic acid molecule operably coupled to a promoter effective for expression of the nucleic acid molecule in the genetically-modified cells, said isolated cellular population obtained as a subpopulation of cells from an *in vitro* differentiation of stem cells;
b) contacting the isolated cellular population with the compound; and
c) assessing an effect of the compound on cells of the isolated cellular population; and wherein said introduced nucleic acid is not used for isolation of the isolated cellular population.

11. A method according to claim 10, wherein said isolated cellular population having genetically modified cells is obtained by:
providing stem cells genetically modified to contain said nucleic acid molecule operably coupled to said promoter;
differentiating the stem cells; and
isolating the isolated cell population from cells differentiated from said stem cells.

12. A method according to claim 9, wherein the isolated cellular population having genetically modified cells is obtained by:
providing stem cells;
differentiating the stem cells;
isolating the isolated cell population from cells differentiated from the stem cells; and
genetically modifying cells of the isolated cell population to contain the nucleic acid molecule operably coupled to the promoter.

13. A method according to any of claims 10-12, wherein said isolating is based upon a selection marker specific for the isolated cellular population.

14. A method for screening for a target nucleic acid comprising the steps of:
a) providing an isolated cellular population having cells genetically modified to contain differing ones of a set of nucleic acid molecules operably coupled to a promoter effective for expression of the nucleic acid molecule in the cells, said isolated cellular population obtained as a subpopulation of cells from an *in vitro* differentiation of stem cells; and
b) assessing the isolated cellular population to identify a target nucleic acid.

15. A method according to claim 14, wherein said isolated cellular population is obtained by:
providing stem cells genetically modified to contain differing ones of the set of nucleic acid molecules;
differentiating the stem cells; and
isolating the isolated cell population from other cells differentiated from the stem cells.

16. A method according to claim 14, wherein said isolated cellular population is obtained by:
providing stem cells;
differentiating the stem cells;
isolating the isolated cell population from other cells differentiated from the stem cells; and
genetically modifying cells of the isolated cell population to contain differing ones of the set of nucleic acid molecules.

17. A method according to any of claims 14-16, wherein the set of nucleic acid molecules is a genomic library, sense cDNA library, an antisense cDNA library, a mixed sense/antisense library, or a RNA interference (RNAi) library.

18. A method for assessing an effect of a compound on the transcriptional activity of a target gene, comprising the steps of:
a) providing an isolated cellular population having genetically-modified cells containing at least one introduced nucleic acid encoding a reporter molecule operably coupled to a promoter for the target gene, said isolated cellular population obtained as a subpopulation of cells from an *in vitro* differentiation of stem cells;
b) contacting the isolated cellular population with the compound; and
c) assessing an effect of the compound on the isolated cellular population based on the amount and/or activity of the reporter molecule.

19. A method according to claim 18, wherein said isolated cellular population is obtained by:
providing stem cells genetically modified to contain at least one introduced nucleic acid encoding a reporter molecule operably coupled to a promoter for the target gene;
differentiating the stem cells; and
isolating the isolated cell population from other cells differentiated from the stem cells.

20. A method according to claim 18, wherein said isolated cellular population is obtained by:
providing stem cells;
differentiating the stem cells;
isolating the isolated cell population from other cells differentiated from the stem cells; and
genetically modifying cells of the isolated cell population to contain at least one introduced nucleic acid encoding a reporter molecule operably coupled to a promoter for the target gene .

21. A genomic validation and drug discovery program, comprising the steps of:
a) screening for a genomic target by assessment of a first isolated cellular population having cells genetically modified to express differing ones of a set of nucleic acid molecules, said set of nucleic acid molecules containing candidate genomic targets;
b) identifying and/or validating the genomic target by assessment of a second isolated cellular population having genetically-modified cells containing said genomic target operably coupled to a promoter effective for expression of the genomic target in the genetically-modified cells; and
c) assessing an effect of a compound on the transcriptional activity of the validated genomic target by contacting with the compound a third isolated cellular population having genetically-modified cells containing an introduced nucleic acid encoding a reporter molecule operably coupled to a promoter for the target gene;
wherein at least one of said first, second and third isolated cellular populations is obtained as a subpopulation of cells from an *in vitro* differentiation of stem cells.

22. A method according to claim 21, wherein said first, second and third isolated cellular populations are all obtained as subpopulations of cells from *in vitro* differentiations of stem cells.

23. Use of an isolated cellular population obtained as a subpopulation of cells from an *in vitro* differentiation of stem cells, in a genomic target screening, identification and/or validation method, or in a compound screening method based on a genomic target or a target gene promoter.

24. A method for assessing the effect of a compound comprising the steps of:
a) providing an isolated cellular population; said isolated cellular population obtained as a subpopulation of cells from an *in vitro* differentiation of stem cells;
b) contacting the isolated cellular population with the compound; and
c) assessing an effect of the compound on cells of the isolated cellular population.

25. An isolated cellular population having genetically-modified cells containing a nucleic acid molecule having genomic target, said genomic target operably coupled to a promoter effective for expression of the genomic target in the genetically-modified cells, said isolated cellular population obtained as a subpopulation of cells from an *in vitro* differentiation of stem cells.

26. A stem cell population having genetically-modified cells containing a nucleic acid molecule including a sequence of nucleotides encoding a target biomolecule, said target biomolecule having a confirmed functional association with a physiological and/or disease or other pathophysiological process.

27. An isolated cellular population having cells genetically modified to contain differing ones of a set of nucleic acid molecules operably coupled to a promoter effective for expression of the nucleic acid molecules in the cells, said isolated cellular population obtained as a subpopulation of cells from an *in vitro* differentiation of stem cells.

28. A stem cell population having cells genetically modified to contain differing ones of a set of a nucleic acid molecules.

29. The stem cell population of claim 28, wherein the set of nucleic acid molecules is a genomic library, sense cDNA library, an antisense cDNA library, a mixed sense/antisense library, or a RNA interference (RNAi) library.

30. An isolated cellular population having genetically-modified cells containing at least one introduced nucleic acid encoding a reporter molecule operably coupled to a promoter for a genomic target, said isolated cellular population obtained as a subpopulation of cells from an *in vitro* differentiation of stem cells.

31. An isolated cellular population of claim 30, obtained by:
providing stem cells genetically modified to contain at least one introduced nucleic acid encoding a reporter molecule operably coupled to a promoter for the genomic target;
differentiating the stem cells; and
isolating the isolated cellular population from other cells differentiated from the stem cells.

32. An isolated cellular population of claim 30, obtained by:
providing stem cells;
differentiating the stem cells;
isolating the isolated cellular population from other cells differentiated from the stem cells; and
genetically modifying cells of the isolated cell population to include at least one introduced nucleic acid encoding a reporter molecule operably coupled to a promoter for the genomic target.

33. A stem cell population having genetically-modified stem cells containing at least one introduced nucleic acid encoding a reporter molecule operably coupled to a promoter for a genomic target.

34. A stem cell population of claim 33, wherein said stem cells also contain an introduced selection marker effective for isolating a subpopulation of cells differentiated from the stem cells.
